# EUROPÄISCHE PATENTANMELDUNG

(11) **EP 2 359 682 A1**
(43) Veröffentlichungstag der Anmeldung: **24.08.2011**
(21) Anmeldenummer: 10153388.3
(22) Anmeldetag: 11.02.2010
(51) Int. Cl.: A01G 33/00, C12M 1/00

(54) **Fassadenelement, Fassadenkonstruktion und Gebäude**

(71) Anmelder: Ove Arup and Partners International Limited, London W1T 4BQ (GB); SSC Strategic Science Consult GmbH, 22761 Hamburg (DE)
(72) Erfinder: Wurm, Jan, 69198 Schriesheim (DE); Kerner, Martin, 22761 Hamburg (DE)
(74) Vertreter: UEXKÜLL & STOLBERG

(57) **Zusammenfassung**

Die vorliegende Erfindung bezieht sich auf ein Fassadenelement mit einem plattenförmigen Bioreaktor (5) zur Kultivierung phototropher Organismen, der ein lichtdurchlässiges plattenförmiges Gehäuse aufweist, in dessen Innerem ein Reaktorraum zur Aufnahme der phototrophen Organismen ausgebildet ist. Ferner bezieht sich die Erfindung auf eine Fassadenkonstruktion (2) und auf ein Gebäude (1), die mindestens ein derartiges Fassadenelement (4) aufweisen.

## Beschreibung

Die vorliegende Erfindung betrifft ein Fassadenelement, eine Fassadenkonstruktion, die mindestens ein derartiges Fassadenelement aufweist, und ein Gebäude mit mindestens einem derartigen Fassadenelement oder einer derartigen Fassadenkonstruktion.

Gebäude weisen regelmäßig Fassadenkonstruktionen auf, die eine äußere Schale bereitstellen, durch die ein Schutz der hinter der äußeren Schale angeordneten Gebäudeteile vor verschiedenen Witterungseinflüssen, wie zum Beispiel Wasser und Wind, sowie Licht, Wärme und/oder Kälte bereitgestellt wird. Ferner spielen die Fassadenkonstruktionen eine Rolle beim Schallschutz des Gebäudeinneren.

Eine übliche, zweischalige Fassadenkonstruktion umfasst eine Außenschale, die in einem Abstand zu der eigentlichen Außenwand bzw. Gebäudehülle des Gebäudes angeordnet ist. Durch eine derartige zweischalige Ausbildung kann ein unerwünschter Wärmeverlust zwischen dem Gebäudeinneren und der Umgebung des Gebäudes verringert werden. Ferner kann in dem Zwischen- oder Pufferraum zwischen der inneren, durch die Außenwand des Gebäudes gebildeten Schale bzw. Gebäudehülle und der Außenschale eine ― ggf. durch den Kamineffekt verstärkte - natürliche Luftströmung entstehen, die erwärmte Luftmassen aus dem Zwischenraum abführt und so zur natürlichen Kühlung beiträgt. Die abgeführte Wärme kann in geeigneter Weise dem Gebäude wieder zugeführt werden.

Die Art der Fassadenkonstruktion hat daher einen entscheidenden Einfluss auf die Belüftung sowie den zur Aufrechterhaltung einer bestimmten Temperatur erforderlichen Energieeinsatz und insgesamt auf die gesamte energetische Bilanz eines Gebäudes.

Ferner ist es bekannt, an einer Fassade rollladen-, jalousien- oder markisenartige Elemente einzusetzen, durch die der Gewinn von Sonnenlicht und -energie kontrolliert werden kann, um einen Sonnen- und/oder Wärmeschutz für Räume bereitzustellen.

Schließlich ist es bekannt, Solarzellen und/oder Sonnenkollektoren insbesondere auf Dächern, aber auch an Fassaden anzuordnen, um für das Gebäude Energie zu erzeugen. Allerdings sind diese Elemente bereits aufgrund ihrer physikalischen Eigenschaften nicht an jeder Stelle einer Fassade einsetzbar. So sind sowohl Solarzellen als auch Sonnenkollektoren lichtundurchlässig und können nur in Bereichen eingesetzt werden, in denen eine vollständige Abschattung der hinter ihnen liegenden Gebäudebereiche kein Problem darstellt. Weiterhin können sie ihren Zweck einer effizienten Energieerzeugung nur erfüllen, wenn sie so angeordnet ist, dass über einen möglichst langen Zeitraum eine hohe Lichteinstrahlung erfolgt. Bereits bei Halbverschattung oder diffusem Licht geht die Leistung derartiger Module gegen Null. Daher ist ein wirtschaftlicher Betrieb zumindest in gemäßigten Breiten (z.B. Nordeuropa) regelmäßig auf Hausdächer beschränkt.

Dies hat zur Folge, dass verschiedenartige Fassadenelemente eingesetzt werden müssen, um alle geforderten und gewünschten Funktionalitäten erfüllen zu können.

Vor diesem Hintergrund besteht das Bestreben, Fassadenkonstruktionen so auszugestalten, dass die energetische Bilanz möglichst optimal ist und gleichzeitig in einfacher Weise verschiedene Funktionalitäten erfüllt werden können.

Es ist Aufgabe der vorliegenden Erfindung, ein Fassadenelement, eine Fassadenkonstruktion und ein Gebäude anzugeben, durch die diese Anforderungen erfüllt werden.

Diese Aufgabe wird durch ein Fassadenelement mit den Merkmalen des Anspruchs 1, durch eine Fassadenkonstruktion mit den Merkmalen des Anspruchs 9 und durch ein Gebäude mit den Merkmalen des Anspruchs 11 gelöst. Bevorzugte Ausführungsformen des Fassadenelements, der Fassadenkonstruktion und des Gebäudes sind Gegenstand der jeweils zugehörigen Unteransprüche.

Nach der vorliegenden Erfindung ist vorgesehen, dass ein Fassadenelement für ein Gebäude, d.h. zur Befestigung an einem Gebäude oder, zum Beispiel bei seiner Erbauung, zur Integration in ein Gebäude, einen plattenförmigen Bioreaktor zur Kultivierung phototropher Zellen und/oder Organismen aufweist. Der Bioreaktor hat ein lichtdurchlässiges plattenförmiges Gehäuse, in dessen Innerem ein Reaktorraum zur Aufnahme der phototrophen Organismen ausgebildet ist. Durch die plattenförmige Ausgestaltung ist das Gehäuse flach und auf zwei gegenüberliegenden Seiten von je einer Seitenwand mit einer im Verhältnis zur Dicke sehr viel ausgedehnteren bzw. größeren Fläche begrenzt, wobei diese beiden großen bzw. größeren Seitenwände durch eine oder mehrere kleine bzw. kleinere Seitenwände verbunden sind. Bevorzugte Bioreaktoren weisen Gehäuse auf, bei denen die großen Seitenwände eine Höhe von 2 bis 4 m und eine Breite von ca. 1 m aufweisen. Um das unerwünschte Absetzen von Organismen an den Reaktorraumwänden zu erschweren, kann es vorteilhaft sein, einzelne oder alle der Ecken und/oder Kanten des Bioreaktorgehäuses abgerundet auszubilden. In einer vorteilhaften Ausgestaltung der Bioreaktoren sind Mittel vorgesehen, durch die während des Betriebs des Bioreaktors innerhalb des im Reaktorraum angeordneten Kulturmediums eine Turbulenz erzeugt wird, die ausreicht, um einem Absetzen oder Anheften der Organismen an den Reaktoroberflächen entgegenzuwirken oder zu verhindern. Dabei ist es besonders vorteilhaft, wenn die Turbulenz ausreichend groß gewählt ist, um einen Transport der Organismen mit Zykluszeiten in der Größenordnung von Millisekunden zwischen Bereichen benachbart zu der lichtzugewandten großen Seitenwand des Gehäuses und Bereichen im Zentrum des Reaktorraums zu transportieren. Ein bevorzugter Bioreaktor, der diese Anforderung erfüllt und bei dem die Turbulenz durch Einbringen von Gasblasen in das Kulturmedium erzeugt wird, wird weiter unten im Detail beschrieben.

Phototrophe Organismen, zu denen ein- und wenigzellige Mikroorganismen wie beispielsweise Algen, Cyanobakterien und pflanzliche Zellkulturen gehören, zeichnen sich dadurch aus, dass sie mittels Photosynthese Licht als Energiequelle nutzen und damit Biomasse aufbauen. Ihre Kultivierung erfolgt in auch als Photobioreaktoren bezeichneten Bioreaktoren mit dem Ziel, sie selbst (Biomasse) oder Stoffwechselprodukte von ihnen zu produzieren. Im Betrieb werden in dem Reaktorraum eines Bioreaktors die jeweiligen Organismen in einem Kultur- bzw. Nährmedium angeordnet, und anschließend wird Licht durch das Reaktorgehäuse in den Reaktorraum eingestrahlt.

Unter phototrophen Organismen werden vorliegend insbesondere Mikroalgen verstanden. Mikroalgen umfassen wie vorliegend verwendet einzellige oder mehrzellige Organismen aus der Gruppe der eukaryotischen Algen oder aus der Gruppe der prokaryontischen Cyanobakterien, die zwischen 1 µm und 100 µm groß sind. Als Algen werden eukaryontische Organismen verstanden, die zu den Pflanzen zählen. Algen, und insbesondere Mikroalgen, werden industriell für die Produktion verschiedener biologischer Substanzen, wie beispielsweise Polysaccharide, Fettsäuren, Proteine, Lipide, Farbstoffe, Vitamine und Sterole, verwendet. Für die Produktion von Fettsäuren und Ölen werden bevorzugt Mikroalgen verwendet, die Chlorophyll a und c besitzen und Lipide statt Stärke als Speicherstoffe aufbauen. Solche Mikroalgen sind bevorzugt in den taxonomischen Gruppen der Heterokontophyta, Dinophyceae, Cyanophyceen, Haptophyta zu finden. Biomassen aus Mikroalgen findet darüber hinaus Anwendung als Futterstoff. Vorzugsweise handelt es sich bei den Mikroalgen, die mit Hilfe der erfindungsgemäßen Verfahren und Bioreaktoren kultiviert werden sollen, um solche der Gattungen Phaeodactylum, Isochrysis, Monodus, Porphyridium, Spirulina, Chlorella, Botryococcus, Cyclotella, Nitzschia, Dunaliella und/oder Nannochloropsis.

Bei den Cyanobakterien handelt es sich um prokaryotische Organismen, die in der Lage sind, oxygene Photosynthese zu betreiben. Cyanobakterien weisen neben Chlorophyll weitere lichtadsorbierenden Pigmente aus der Gruppe der Phycobiline, die ihnen eine im Vergleich zu Pflanzen optimalere Lichtnutzung ermöglichen. Aus diesem Grund können Cyanobakterien Schwachlichtbereiche besiedeln, in die Pflanzen aufgrund der auf Chlorophyll basierenden Photosynthesesysteme nicht vordringen können. Die vorliegenden Bioreaktoren und Verfahren können prinzipiell mit beliebigen Arten von Cyanobakterien betrieben werden. Vorzugsweise handelt es sich bei den zu kultivierenden Cyanobakterien um solche der Gattungen, Acaryochloris, Anabaena, Aphanizomenon, Chroococcus, Gloeobacter, Gloeocapsa, Lyngbya, Microcystis, Microcoleus, Nodularia, Nostoc, Oscillatoria, Phormidium, Prochlorococcus, Prochloron, Prochlorothrix, Scytonema, Spirulina, Stigonema, Synechococcus und/oder Trichodesmium.

Ferner lassen sich auch pflanzliche Zellkulturen und Moose in den Bioreaktoren anzüchten.

In diesem Zusammenhang besteht der Vorteil, dass Bioreaktoren eine Kultivierung in einem nach aussen abgedichteten System mit in sich geschlossenen Gas- und Wasserkreisläufen ermöglichen. Auf diese Weise kann zuverlässig verhindert werden, dass Mikroorganismen ins Freiland entweichen. Dies ist eine Voraussetzung, um gentechnisch modifizierte Organismen ohne ein umhüllendes Containment im Freiland kultivieren zu können.

Der Bioreaktor bzw. sein Gehäuse ist bevorzugt quaderförmig. Demnach weist das Gehäuse bevorzugt neben den zwei gegenüberliegenden Seitenwänden mit einer im Verhältnis zur Dicke sehr viel ausgedehnteren bzw. größeren Fläche zwei weitere einander gegenüberliegende Seitenwände mit einer kleineren Fläche und zwei einander gegenüberliegende Stirnwände auf. Aufgrund der plattenförmigen Ausgestaltung des Gehäuses weist jede der zuletzt genannten vier Gehäusewände eine Fläche auf, die sehr viel kleiner bzw. weniger ausgedehnt als diejenige der zuerst genannten ausgedehnten, größeren bzw. großen Seitenwände ist. Der Abstand zwischen den kleineren bzw. kleinen Seitenwänden definiert die Breite des Reaktorraums, der Abstand zwischen den Stirnwänden definiert die Länge des Reaktorraums, und der Abstand zwischen den größeren Seitenwänden definiert die Dicke bzw. Tiefe oder Höhe des Reaktorraums.

In jedem Fall erfolgt der Eintrag von Licht in den Reaktorraum zumindest auch durch eine oder beide der größeren Seitenwände des plattenförmigen Gehäuses. In diesem Zusammenhang ist es möglich, vorzusehen, dass das Gehäuse lediglich teilweise aus einem lichtdurchlässigen Material hergestellt ist, das zumindest einen Teil der großen Seitenwände oder bevorzugt die gesamten solchen großen Seitenwände bildet. Es ist jedoch bevorzugt, dass auch weitere Teile des Gehäuses und bevorzugt das gesamte oder im wesentlichen das gesamte Gehäuse aus einem lichtdurchlässigen Material ausgebildet ist. Vorteilhafte lichtdurchlässige Materialien sind lichtdurchlässige, durchsichtige oder glasklare Kunststoffmaterialien, wie zum Beispiel PET, mit einer hohen Durchlässigkeit, insbesondere für die photosynthetisch aktive Strahlung zwischen 400 und 700 nm. Es ist bevorzugt, dass die Durchlässigkeit in diesem photosynthetisch wirksamen Spektralbereich mindestens 90% beträgt.

Es ist möglich, für das Fassadenelement vorzusehen, dass es unmittelbar in ein Gebäude integriert wird, indem es zum Beispiel fest in eine Außenwand eingebaut wird. Es ist jedoch bevorzugt, dass das Fassadenelement ferner eine Befestigungseinrichtung aufweist, mit deren Hilfe der Bioreaktor an einer Außenseite eines Gebäudes angebracht bzw. befestigt werden kann. Dies kann über eine je nach vorgesehenem Anwendungsfall geeignete Ausgestaltung der Befestigungseinrichtung erfolgen, indem der Bioreaktor bzw. das Fassadenelement an einer bestehenden Gebäudehülle, wie zum Beispiel einer Außenwand oder einem Dach, befestigt wird oder indem das Fassadenelement selbst als Teil einer solche Gebäudehülle zu deren Aufbau verwendet wird. In letzterem Fall wird zum Beispiel erst unter anderem durch die Fassadenelemente eine Außenwand oder ein Dach hergestellt. Die Befestigungseinrichtung kann zum Beispiel ein geeignetes Gestell oder eine geeignete Träger- oder Unterkonstruktion umfassen, die an dem Bioreaktor befestigt oder einstückig mit diesem ausgebildet ist. Ferner ist es möglich, dass die Befestigungseinrichtung teilweise oder vollständig integral mit der Gebäudehülle bzw. der Gebäude ausgebildet ist. So könnten beispielsweise Träger, Schienen oder Mauervorsprünge zur Befestigung des Fassadenelements an der Gebäudehülle ausgebildet oder an dieser befestigt sein. Für eine Fassade, die mehrere erfindungsgemäße Fassadenelemente aufweist, ist es auch möglich, dass sich mehrere Fassadenelemente eine Befestigungseinrichtung teilen, die dann beispielsweise in Form einer Gerüst- oder Schienenkonstruktion ausgebildet sein kann, an der die einzelnen Bioreaktoren befestigt sind. Im einfachsten Fall kann die Befestigungseinrichtung Bohrungen in dem Fassadenelement oder dem Bioreaktorgehäuse und Schrauben oder Bolzen umfassen.

Es ist möglich, für das Fassadenelement vorzusehen, dass es in einer fixierten bzw. statischen Position und Stellung an einem Gebäude bzw. als Teil einer Fassadenkonstruktion angeordnet ist. Es ist jedoch bevorzugt, wenn für das Fassadenelement eine Verstelleinrichtung vorgesehen ist, mit der nach Befestigung des Bioreaktors an einer Außenseite eines Gebäudes oder einer Gebäudehülle eine Position und/oder eine Winkelstellung des Bioreaktors in Bezug auf das Gebäude verändert bzw. eingestellt werden kann. Dabei ist es möglich, dass die Verstelleinrichtung passiv ist, d.h. dass der Bioreaktor manuell oder durch einen separaten Antrieb bewegt werden muss und mit Hilfe der Verstelleinrichtung in einer gewünschten Winkelstellung bzw. Position fixiert werden kann. Es ist jedoch bevorzugt, dass die Verstelleinrichtung so ausgestaltet ist, dass sie betrieben werden kann, um den Bioreaktor aktiv zu bewegen und in einer gewünschten Winkelstellung bzw. Position zu fixieren. Die Verstelleinrichtung kann z.B. an dem Bioreaktor befestigt oder integral mit diesem ausgebildet sein. Ferner ist es möglich, dass die Verstelleinrichtung teilweise oder vollständig integral mit der Gebäudehülle bzw. dem Gebäude ausgebildet ist. Ist eine Befestigungseinrichtung vorgesehen, so kann die Verstelleinrichtung als integraler Bestandteil der Befestigungseinrichtung ausgestaltet sein. Wie weiter unten ausgeführt wird kann durch eine solche Verstelleinrichtung die Funktionalität des Fassadenelements noch mehr erweitert werden.

Wird ein Fassadenelement mit der oben beschriebenen Ausgestaltung außen an einem Gebäude bzw. als Teil der Außenschale eines Gebäudes eingesetzt, bevorzugt in einer Ausrichtung ungefähr nach Süden, ergibt sich der Vorteil, dass es nicht nur als reguläres Fassadenelement, das einen Witterungsschutz vor Wind und Schlagregen gewährleistet, ggf. eine geländerförmige Absturzsicherung bereitstellt, einen Wärmeverlust zwischen dem Gebäudeinneren und der Umgebung reduziert und als Schallschutz fungiert, verwendet werden kann, sondern - ggf. in Abhängigkeit von der Positionierung an der Außenwand und in Bezug auf etwaige Fenster oder Türen - flexibel und dynamisch gezielt anpassbar mehrere Zusatzfunktionalitäten bereitstellen kann. Es sei in diesem Zusammenhang darauf hingewiesen, dass der Begriff "Fassade" im Rahmen dieser Anmeldung auch Dächer umfasst.

So kann die Lichttransmission und damit auch der Energieeintrag von außen in das Gebäude durch geeignete Wahl der Dichte phototropher Organismen in dem Reaktorraum des Bioreaktors, d.h. der Zelldichte bzw. Zellzahl, beeinflusst werden. Da die für einen Betrieb des Bioreaktors minimal erforderliche Dichte der phototrophen Organismen allgemein mit zunehmender Intensität der Sonnenstrahlung zunimmt (bei hoher Intensität kann besteht die Gefahr einer Lichtschädigung der Organismen, der durch eine-gegenseitige Abschattung bei hoher Dichte entgegengewirkt wird), ergibt sich die vorteilhafte Konstellation, dass bei starker Sonnenstrahlung eine hohe Dichte eingestellt werden sollte, die die Lichttransmission stark vermindert und im Idealfall nahezu auf Null absenkt und auf diese Weise eine Aufheizung des Gebäudes vermindert bzw. - bei entsprechender Verwendung - einen hohen Sonnenschutz bereitstellt, während bei schwacher Sonnenstrahlung die Dichte bzw. die Lichttransmission nach Bedarf niedrig gewählt werden kann, so dass mehr Licht bzw. Wärme das Gebäude erreichen.

Insgesamt ist es in Abhängigkeit der Dichte phototropher Organismen und der Dicke des Bioreaktors möglich, wahlweise zwischen 5 und 98% des eingestrahlten Sonnenlichtes zu absorbieren. Damit besteht die Möglichkeit, das Lichtklima von Gebäuden in gewünschter Weise zu regulieren.

Ist eine Verstelleinrichtung vorgesehen, wie sie oben beschrieben wurde, so ist es aufgrund der Veränderbarkeit der Winkelstellung der Bioreaktoren zunächst möglich, mit Hilfe eines erfindungsgemäßen Fassadenelements, das einen Teil einer beabstandet von einer Gebäudehülle bzw. einer eigentlichen Außenwand oder eines eigentlichen Dachs angeordneten Außenschale einer Gebäudefassade bildet, eine gezielte Belüftung des Zwischenraums zwischen der Gebäudehülle und der Außenschale zu bewirken und auf diese Weise den Wärmeaustausch zwischen dem Gebäudeinnerem und der Umgebung nach Bedarf zu beeinflussen, wodurch die Behaglichkeit innerhalb des Gebäudes verbessert werden kann. Ferner bietet die Veränderbarkeit der Winkelstellung der Bioreaktoren und/oder ihrer Position auch die Möglichkeit, diese insbesondere bei geeigneter Positionierung in der Nähe eines Fensters oder einer Tür, bevorzugt oberhalb des Fensters bzw. der Tür, als variabel veränderbaren Sonnenschutz zu verwenden oder eine gezielte Tageslichtlenkung in das Gebäudeinnere zu erzielen.

Diese vorteilhaften Funktionalitäten sind in jedem Fall gekoppelt mit der Erzeugung von Energie, die von einem Gebäude genutzt werden kann, an dem das Fassadenelement angebracht ist, und somit die Energiebilanz des Gebäudes verbessert. In diesem Zusammenhang ist zunächst die Produktion von Biomasse zu berücksichtigen. Die Biomasse kann beispielsweise durch eine Anlage zur hydrothermalen Konversion innerhalb des Gebäudes oder durch biologische Fermentation in einer diesem zugeordneten Biogasanlage in Methan oder ein anderes Biogas (z.B. H₂) umgewandelt werden. Biogas bzw. Methan können dann in das Erdgasnetz eingespeist werden und/oder im Gebäude für die Energieerzeugung verwendet werden. Eine weitere Möglichkeit der hydrothermalen Konversion ist die Umwandlung der Biomasse zu Ölen, die als Wertstoff zur Gewinnung von Feinchemikalien oder wie das Biogas als Brennstoff weiterverwendet werden können. Darüber hinaus wirken Bioreaktoren wie Sonnenkollektoren, d.h. im Reaktorraum befindliches Kulturmedium wird erwärmt. Die Effizienz nimmt bei plattenförmigen Bioreaktoren mit zunehmenden Zelldichten von phototrophen Organismen zu. Bei einer Zelldichte entsprechend einem Trockengewicht von etwa 20 g pro Liter kann der Wärmeertrag bis zu 75% von demjenigen in herkömmlichen Solarkollektoren gleicher Fläche betragen. In diesem Zusammenhang ist wieder zu beachten, dass die Bioreaktoren den Vorteil besitzen, dass sie bei starker Sonnenstrahlung mit hohen Dichten phototropher Organismen betrieben werden sollten, bei denen die Effizienz des Betriebs als Sonnenkollektoren besonders hoch ist, während bei geringer Sonnenstrahlung die Dichte der phototrophen Organismen nach Bedarf abgesenkt werden kann.

Aufgrund der Erwärmung des Kulturmediums, das eine Folge der Absorption von Sonnenlicht ist, gibt der Bioreaktor je nach Temperatur des Kulturmediums und Temperatur des umgebenden Mediums, Energie ab oder nimmt Energie auf. Dieser Effekt kann beispielsweise während der Heizperiode gezielt dazu verwender werden, einen ggf. zwischen dem Bioreaktor und der Gebäudehülle angeordneten Luftraum zu erwärmen, um so die Wärmeverluste des Gebäudes zu reduzieren ("aktive Wärmedämmung"). Im Sommer hingegen kann durch die Aufnahme von Energie aus dem umgebenden Medium (Außenluft) durch die Bioreaktoren ein Kühleffekt an der Gebäudehülle erzielt werden. Die von dem Photobioreaktor aufgenommene Wärme kann durch geeignete Vorrichtungen gespeichert und auf andere Weise dem Gebäude wieder zugeführt werden.

In Kombination mit den obigen Funktionalitäten hat die hohe Energieerzeugung durch Biomasseproduktion und gleichzeitige Umwandlung von Licht in Wärme einzigartige Eigenschaften von plattenförmigen Bioreaktoren als Fassadenelemente zur Folge. Dabei ist es möglich, dass sie herkömmliche Fassadenelemente vollständig ersetzen.

In einer bevorzugten Ausführungsform mit einer Befestigungseinrichtung ist die Befestigungseinrichtung in der Weise ausgestaltet, dass der Bioreaktor, nachdem er mit Hilfe der Befestigungseinrichtung an einem Gebäudehüllenabschnitt befestigt worden ist, von dem Gebäudehüllenabschnitt beabstandet ist und bevorzugt parallel zu diesem verläuft oder in eine parallele Ausrichtung gebracht werden kann, so dass sich zwischen dem Bioreaktor und dem Gebäudehüllenabschnitt ein Zwischenraum befindet. In einer alternativen bevorzugten Ausführungsform weist das Fassadenelement neben dem Bioreaktor selbst einen Gebäudeschalenabschnitt auf, an dem der Bioreaktor in der Weise befestigt ist, dass er von dem Gebäudehüllenabschnitt beabstandet ist und sich zwischen dem Bioreaktor und dem Gebäudehüllenabschnitt ein Zwischenraum befindet, wobei der Bioreaktor bevorzugt parallel zu dem Gebäudehüllenabschnitt verläuft oder in eine parallele Ausrichtung gebracht werden kann. Ein Beispiel für eine solche Ausgestaltung eines Fassadenelements, das erst zum Aufbau einer Gebäudehülle verwendet wird, ist ein Kastenfenster, dessen äußere Verkleidung dann durch den Bioreaktor gebildet wird. In beiden Ausführungsformen können die Zwischenräume je nach angestrebter Funktionalität zwischen 1 cm und mehreren Metern betragen. Im Falle eines größeren Abstands wird zwischen Fassade und Gebäudeaussenwand ein Raum gebildet, der u.a. als Wohn- oder Lagerraum genutzt werden kann.

In diesen beiden Ausführungsformen ist es ferner bevorzugt, wenn eine Verstelleinrichtung, wie sie oben beschrieben wurde, vorgesehen und derart ausgestaltet ist, dass der Bioreaktor wahlweise in eine erste Stellung, in der sich der Bioreaktor parallel zu dem Gebäudehüllenabschnitt erstreckt, und in mindestens eine zweite Stellung gebracht werden kann, in der der Bioreaktor relativ zu dem Gebäudehüllenabschnitt schräg verläuft. Mit anderen Worten kann das Fassadenelement, zum Beispiel mit Hilfe der Befestigungseinrichtung oder durch direkte Integration eines Gebäudehüllenabschnitts des Fassadenelements in die Gebäudehülle, bestimmungsgemäß an einer Außenseite eines Gebäudes, wie zum Beispiel an einer Außenwand oder einem Dach des Gebäudes, angebracht werden, und anschließend kann der Bioreaktor sowohl in paralleler als auch in nicht paralleler Ausrichtung zu der Außenseite, d.h. z.B. der Außenwand bzw. dem Dach, eingestellt werden. Dabei kann für erfindungsgemäße Fassadenelemente, die lediglich die Funktion eines üblichen Fassadenelements erfüllen, die parallele Ausrichtung beispielsweise die Normalposition sein, aus der der Bioreaktor nach Bedarf - wie oben bereits dargestellt - heraus bewegt wird. In der parallelen Ausrichtung bildet der Bioreaktor üblicherweise einen Teil einer durchgehenden Außenschale des Gebäudes. In dieser Stellung können die höchsten Effizienzen in Bezug auf die Funktionalitäten Wärmedämmung und Schallschutz erreicht werden.

In dieser Ausführungsform ist es besonders bevorzugt, wenn die Verstelleinrichtung in der Weise ausgestaltet ist, dass der Bioreaktor nach Befestigung an der Außenseite eines Gebäudes aus der ersten Stellung um mindestens bis zu 60° um eine horizontale Achse und/oder um mindestens bis zu 30° um eine dazu senkrechte Achse gedreht werden kann, d.h. wenn eine Winkelvariation diskret oder kontinuierlich in diesen Bereichen möglich ist. Bei einer in Bezug auf den Erdboden senkrecht verlaufenden Außenwand wären dies zum Beispiel die horizontale Achse und die vertikale Achse. Diese Winkelbereiche ermöglichen, zumindest bei einer ungefähr nach Süden ausgerichteten Außenwand, zum einen eine über den gesamten Tagesverlauf praktisch ausreichende Möglichkeit der Nachführung mit der Sonne und einen damit verbundenen im Idealfall senkrechten Einfallwinkel für die Einstrahlung, mit entsprechend hoher Biomasseproduktion bzw. hohem Wärmeeintrag, und zum anderen eine zur Bereitstellung eines variablen Sonnenschutzes vorteilhafte Einstellbarkeit.

Ist eine Verstelleinrichtung vorgesehen, so ist es von Vorteil, wenn die Verstelleinrichtung einen Motor aufweist, der mit dem Bioreaktor in der Weise gekoppelt ist, dass er den Bioreaktor zur Veränderung der Position und/oder Winkelstellung bewegen kann. Dadurch ist sowohl eine einfache und jederzeit vornehmbare Anpassbarkeit der Fassadenfunktionalitäten durch einen Benutzer möglich, als auch eine Automatisierung der Energiegewinnung und der Anpassung der verschiedenen Funktionalitäten in Abhängigkeit von verschiedenen Parametern, wie zum Beispiel der Tageszeit, der Jahreszeit, dem Stand der Sonne, der Temperatur in dem Bioreaktor und/oder der Gebäudetemperatur.

In jedem Fall ist es in Ausführungsformen mit einer Verstelleinrichtung bevorzugt, wenn dem Fassadenelement eine Winkelstellungs- und/oder Positionssteuereinrichtung zugeordnet ist, die angepasst ist, um im Betrieb die Winkelstellung und/oder die Position des Bioreaktors, bevorzugt automatisch, in Bezug auf das Gebäude in Abhängigkeit von dem Stand der Sonne, der Gebäudetemperatur, der Reaktortemperatur, der Jahreszeit und/oder der Tageszeit zu verändern. Zu diesem Zweck können geeignete Sensoren vorhanden sein. Eine entsprechende Winkelstellungs- und/oder Positionssteuereinrichtung kann in jedem Fassadenelement separat vorgesehen sein. Es ist aber auch möglich und ggf. von Vorteil, wenn mehrere oder alle erfindungsgemäßen Fassadenelemente, die Teil einer Fassade sind, durch eine einzige Winkelstellungs- und/oder Positionssteuereinrichtung gesteuert werden, die dann z.B. mit geeigneten Motoren der Fassadenelemente verbunden ist.

In einer vorteilhaften Ausgestaltung weist das Fassadenelement einen Anschluss auf, an den eine Wärmetransporteinrichtung angeschlossen werden kann, um Wärme zwischen dem Bioreaktor und einem Gebäude auszutauschen. Dieser Wärmeaustausch kann je nach Bedarf und Steuerung der Bioreaktorparameter den Eintrag von Wärme aus dem Gebäude in den Bioreaktor zu Zwecken der Kühlung des Gebäudes oder einen Wärmetransport in umgekehrter Richtung zur Erwärmung des Gebäudes oder Speicherung von Wärme im Gebäude zur späteren Verwendung umfassen. Zu diesem Zweck kann beispielsweise in vorteilhafter Weise in dem Bioreaktor eine Leitung angeordnet sein, die in Bezug auf den Reaktorraum so angeordnet und ausgestaltet ist, dass im Betrieb zwischen einem die Leitung durchströmenden Fluid und einem in dem Reaktorraum angeordneten Kulturmedium Wärme ausgetauscht werden kann. Dabei sind zwei gegenüberliegende Enden der Leitung mit dem Anschluss verbunden, so dass über den Anschluss Fluid in die Leitung einströmen und aus dieser wieder austreten kann, nachdem es die Leitung durchströmt hat. Mit anderen Worten ist dann nach Anschluss an geeignete Gebäudeleitungen ein Teil eines Kreislaufs eines Wärmetauschers an oder in dem Bioreaktor angeordnet, um Wärme mit dem im Reaktorraum befindlichen Kulturmedium auszutauschen. Die Leitung in dem Bioreaktor kann eine durchgehende Leitung sein, die beispielsweise mäanderförmig geschwungen ausgestaltet ist, oder aber eine Leitung mit mehreren Verzweigungen. Die Leitung kann unmittelbar durch den Reaktorraum verlaufen, oder es kann ein mit dem Reaktorraum verbundener separater Kreislauf vorgesehen sein, über den Wärme zwischen dem Fluid in der Leitung und dem Kulturmedium ausgetauscht wird. Für einen Wärmeeintrag sollte die Temperatur bevorzugt im Primärkreislauf 10 bis 60 °C und im durch das Kulturmedium gebildeten Sekundärkreislauf 2 bis 50 °C, mehr bevorzugt 5 bis 50 °C und am meisten bevorzugt 12 bis 40 °C betragen. Insgesamt ist es bevorzugt, wenn das Kulturmedium im Kreislauf geführt wird und dabei über einen Wärmetauscher Wärme abgeführt bzw. zugeführt wird und so in den Reaktoren ganzjährig Temperaturen in den genannten Bereichen gehalten werden.

In einer bevorzugten Ausgestaltung ist dem Fassadenelement eine Temperatursteuereinrichtung zugeordnet, die angepasst ist, um im Betrieb die Temperatur eines in dem Reaktorraum des Bioreaktors befindlichen Kulturmediums automatisch in Abhängigkeit von der Tageszeit, der Jahreszeit, der in den Reaktorraum einfallenden Lichtintensität und/oder der Temperatur des Kulturmediums zu steuern. Dabei kann jedes einzelne Fassadenelement selbst eine eigene Temperatursteuereinrichtung aufweisen, oder eine Gruppe von Fassadenelementen kann sich eine solche Temperatursteuereinrichtung teilen. Beispielsweise kann eine zentrale Einrichtung vorgesehen sein, die die Temperatur in den Bioreaktoren regelmäßig bzw. ständig über das im Kreislauf aus den Bioreaktoren ab- und nach der Passage durch einen Wärmetauscher zur Wärmeabfuhr oder Wärmezufuhr wieder zugeführte Kulturmedium regelt. Zum Zwecke der Temperatursteuerung kann das Fassadenelement geeignete Sensoren enthalten, oder solche Sensoren können dem Fassadenelement oder einem Kreislaufsystem des Kulturmediums zugeordnet sein. Eine Temperatursteuereinrichtung ermöglicht die Optimierung der Bedingungen zur effizienten Kultivierung phototropher Organismen unter Berücksichtigung der durch die gewünschten Funktionalitäten vorgegebenen Randbedingungen. Bevorzugt sollte im Betrieb die Temperatur des Kulturmediums ganzjährig zwischen 12 und 40 °C gehalten werden, so dass die Temperatursteuereinrichtung bevorzugt angepasst ist, um die Temperatur in diesem Bereich zu halten.

Damit die phototrophen Organismen im Winter nicht absterben, ist es nämlich notwendig, die Temperatur über einer gewissen Temperatur zu halten, die deutlich oberhalb des Gefrierpunktes von Wasser liegt. Darunter können die Organismen die Lichtstrahlungsintensitäten nicht verkraften, wie sie an klaren Wintertagen beispielsweise auch in Nordeuropa auftreten und sterben innerhalb weniger Stunden. Für die Mikroalge Chlorella vulgaris hat sich beispielsweise herausgestellt, dass sie erst oberhalb einer Kultivierungstemperatur von 12 °C hohe Sonneneinstrahlungen unbeschadet überlebt bzw. diese photosynthetisch nutzen kann. In der folgenden Tabelle sind bevorzugte minimale Kultivierungstemperaturen in Abhängigkeit der Strahlungsintensität angegeben:

| Maximale PAR-Strahlung (µmol/m²/s) | min. Kultivierungstemperatur (°C) |
|---|---|
| 100 | 2 |
| 500 | 5 |
| 2500 | 10 |

Für eine effiziente Kultivierung in Nordeuropa ist vor diesem Hintergrund gemäß einer bevorzugten Ausführungsform vorgesehen, dass die Temperatursteuereinrichtung benutzt wird oder angepasst ist, um die Temperatur des Kulturmediums während der Nacht auf mindestens 1 °C zu halten, vor Sonnenaufgang auf mindestens 5 °C zu erhöhen, tagsüber die maximale Temperatur unter einer Temperatur von beispielsweise 50 °C zu halten, bei der die jeweiligen phototrophen Organismen geschädigt werden, und nach Sonnenuntergang wieder auf die bereits genannte Nachttemperatur abzusenken. Die maximale Nachttemperatur liegt bevorzugt in einem Bereich von 1 bis 15 °C und sollte gewährleisten, dass mikrobielle Abbauprozesse reduziert sind.

Es ist bevorzugt, dass dem Fassadenelement bzw. ggf. einem entsprechenden Kreislauf des Kulturmediums eine Transmissionssteuereinrichtung zugeordnet ist, die angepasst ist, um die Dichte der phototrophen Organismen im Reaktorraum des Bioreaktors gezielt zu verändern und auf diese Weise die Lichtdurchlässigkeit des Bioreaktors einzustellen. Dabei kann jedes einzelne Fassadenelement selbst eine eigene Transmissionssteuereinrichtung aufweisen, oder eine Gruppe von Fassadenelementen kann sich eine solche Transmissionssteuereinrichtung teilen. Eine Transmissionssteuerung wird bewirkt, indem die Dichte an phototrophen Organismen im Kulturmedium nach Bedarf konstant gehalten oder geeignet geändert wird, d.h. diese Einstellung kann über die Zelldichte als Solltrockenmasse in g/l erfolgen. Mit anderen Worten kann z.B. das Ausmaß der Lichtabsorption zeitlich über die Zelldichten über entsprechende Verdünnung des Reaktorinhalts mit zellfreiem Kulturmedium erfolgen. Im Tagesverlauf kann auf diese Weise die Lichtabsorption entsprechend der Wachstums- bzw. Zellteilungsrate um bis zu 100% variiert werden.

Wie bereits erläutert worden ist, kann eine automatische und/oder manuelle Einstellung der Transmissionseigenschaften des Bioreaktors in vorteilhafter Weise verwendet werden, um die Gebäudetemperatur in gewünschter Weise zu beeinflussen oder einen Sonnenschutz geeignet anzupassen.

In einer vorteilhaften Ausgestaltung ist die Oberfläche des Bioreaktors - und bevorzugt eine oder beide der großen Seitenwände - vollständig oder zumindest teilweise mit Dünnfilmsolarzellen beschichtet, um die Energieeffizienz weiter zu erhöhen.

Für das Wachstum der phototrophen Organismen ist es von Vorteil, wenn - ggf. zusätzlich zu einem zum Bewirken einer Turbulenz dienenden Gaseintrag - CO₂ in das Kulturmedium eingebracht wird. Zu diesem Zweck können Diffusoren und/oder statische Mischer vorgesehen sein. Die Diffusoren können in vorteilhafter Weise als poröse Schläuche oder Rohre mit einer Porengröße von bevorzugt 5 bis 35 µm ausgebildet sein, die an geeigneter Stelle im Reaktorraum angeordnet sind. Die Diffusoren können einen, beispielsweise durch eine der größeren Seitenwände vorgesehenen Zulauf aufweisen, über den die Beaufschlagung mit Gas erfolgt. Das Gas tritt dann im Betrieb über die Poren als feinste Gasblasen ins Kulturmedium über. Da die Geschwindigkeit des Gasaustausches zwischen Gas und Flüssigkeit von der Größe bzw. der Oberfläche der Gasblasen abhängt, ist dieser umso größer, je kleiner die Gasblasen sind. Die Größe der Poren ist also so zu wählen, dass möglichst kleine Gasblasen entstehen. Bevorzugt sind Porengrößen von < 10 Mikrometern. Bekannte statische Mischer können genutzt werden, um das Gas in einem Rohrleitungssystem im Kulturmedium zu lösen. So ist es zum Beispiel im Fall des Betriebs der Bioreaktoren als Turbidostaten möglich, das im Kreislauf geführte Kulturmedium über statische Mischer mit Gas anzureichern. Der Gasdruck in den Diffusoren kann in vorteilhafter Weise relativ niedrig gewählt werden. Bevorzugte Werte sind 0,1 bis 2 bar Überdruck, mehr bevorzugt 0,4 bis 0,6 bar Überdruck.

Es ist bevorzugt, dass im Betrieb in bestimmten Zeitabständen oder kontinuierlich ein Austausch des Kulturmediums bzw. eine Rückführung im Kreislauf stattfindet. Um diesen Betrieb zu ermöglichen, weist der Bioreaktor bevorzugt einen Zulaufanschluss auf, über den die Flüssigkeit mit Hilfe einer Pumpe in den Reaktorraum gepumpt werden kann, und einen Ablaufanschluss, über den die Flüssigkeit abgegeben wird. Am Ablauf bzw. an anderer Stelle im äußeren Kreislaufabschnitt kann in vorteilhafter Weise durch geeignete Einrichtungen sowohl die Aufnahme von Messparametern als auch die Zudosierung von Nährstoffen und ggf. von Säuren oder Basen zur Einstellung des pH-Wertes im Kulturmedium erfolgen. Darüber hinaus können über die Kreislaufführung Teilströme des Kulturmediums erwärmt oder abgekühlt werden, um - wie oben bereits beschrieben - optimale Temperaturbedingungen in dem Bioreaktor zu erreichen. Eine Erwärmung ist bevorzugt durchzuführen, wenn die Temperatur im Bioreaktor unter 12 °C sinkt, wobei im Teilstrom Temperaturen bis zu 30 °C eingesetzt werden können, und eine Abkühlung sollte erfolgen, wenn die Temperatur im Bioreaktor über 40 C steigt. Es ist bevorzugt, wenn der Volumenstrom des Kulturmediums bei Kreislaufführung in Prozent des Bioreaktorvolumens pro Stunde 0 bis 200% und mehr bevorzugt 50 bis 100% beträgt. Dabei bedeutet ein Wert von 50% zum Beispiel, dass das Bioreaktorvolumen alle zwei Stunden einmal umgewälzt wird.

Ein Fassadenelement mit den oben beschriebenen Eigenschaften kann in vorteilhafter Weise als Teil einer Fassadenkonstruktion eingesetzt werden, die zur Befestigung an einer Außenseite eines Gebäudes, wie zum Beispiel der Gebäudehülle oder Teilen von dieser, wie etwa einer oder mehreren Außenwänden oder dem Dach, vorgesehen ist oder integral mit einem Gebäude ausgebildet ist. Demnach ist es möglich, dass die Fassadenkonstruktion an einer bestehenden Gebäudehülle befestigt wird, wie zum Beispiel einer Außenwand oder einem Dach, oder dass die Fassadenkonstruktion selbst erst zumindest einen Teil der Gebäudehülle bereitstellt. In jedem Fall kann eine solche Fassadenkonstruktion, die nach Befestigung an einer Außenseite eines Gebäudes die Außenschale des Gebäudes bildet, ein oder bevorzugt mehrere erfindungsgemäße Fassadenelemente umfassen. Dann können die jeweiligen Bioreaktoren einen Teil der Außenfläche, wie zum Beispiel an einzelnen Außenwänden oder Dachabschnitten, oder bevorzugt die gesamte oder im wesentlichen die gesamte Außenfläche der Außenschale bereitstellen (Fenster und Türen werden im allgemeinen ausgespart werden). Insbesondere ist es bevorzugt, wenn eine solche Fassadenkonstruktion zwei oder mehr nebeneinander angeordnete erfindungsgemäße Fassadenelemente aufweist, die in Kombination einen durchgehenden Abschnitt der Fassadenkonstruktion bzw. einen durchgehenden Abschnitt der Außenschale des Gebäudes bilden.

Entsprechend den obigen Erläuterungen ist es von Vorteil, wenn die Fassadenkonstruktion ein oder mehrere erfindungsgemäße Fassadenelemente mit einer Verstelleinrichtung aufweist, wie sie oben beschrieben wurde, wobei die Bioreaktoren der Fassadenelemente, z.B. nach Befestigung der Fassadenkonstruktion an einer Gebäudehülle eines Gebäudes, wahlweise mit den entsprechenden Verstelleinrichtungen in eine erste Anordnung, in der die Bioreaktoren parallel zur Gebäudehülle verlaufen und - bei Vorhandensein von mehr als einem entsprechenden Fassadenelement - bevorzugt in Kombination einen Teil einer durchgehenden Außenschale bilden, und in eine zweite Anordnung gebracht werden können, in der zumindest einer der Bioreaktoren in Bezug auf die Gebäudehülle schräg verläuft, um den Eintritt von Luft und/oder Licht durch die Außenschale zu ermöglichen.

Die Bioreaktoren mehrerer erfindungsgemäßer Fassadenelemente können einzeln oder miteinander verbunden als Batch oder als Turbidostat betrieben werden. Als Batch findet ein Austausch des Kulturmediums in bestimmten Zeitabschnitten statt. Als Turbidostat wird das Kulturmedium kontinuierlich über die Zeit ausgetauscht bzw. im Kreislauf zurückgeführt, d.h. es findet ein kontinuierlicher Zu- und Ablauf statt. Um diesen Betrieb zu ermöglichen, weist jeder entsprechende Bioreaktor in einer vorteilhaften Ausführungsform in der oben bereits beschriebenen Weise einen Zulaufanschluss auf, über den die Flüssigkeit mit Hilfe einer Pumpe in den Reaktorraum gepumpt werden kann, und einen Ablaufanschluss, über den die Flüssigkeit abgegeben wird. Sind mehrere Reaktoren in Reihe miteinander verbunden, so genügt es, wenn jeweils nur die Reaktoren am Anfang und Ende der Reihe einen Zulauf bzw. Ablauf aufweisen. Am Ablauf bzw. an anderer Stelle im äußeren Kreislaufabschnitt kann in vorteilhafter Weise durch geeignete Einrichtungen sowohl die Aufnahme von Messparametern als auch die Zudosierung von Nährstoffen und ggf. von Säuren oder Basen zur Einstellung des pH-Wertes im Kulturmedium erfolgen. Darüber hinaus können über die Kreislaufführung Teilströme des Kulturmediums erwärmt oder abgekühlt werden, um - wie oben bereits beschrieben - optimale Temperaturbedingungen in dem Bioreaktor zu erreichen.

Ein Fassadenelement mit der oben dargestellten Ausgestaltung ist dazu vorgesehen, durch Befestigung an einer Außenseite eines Gebäudes bzw. Integration in eine Gebäudehülle an oder in einer Fassade des Gebäudes verwendet zu werden, die dadurch vorteilhafte Eigenschaften aufweist, und eine Fassadenkonstruktion mit der obigen Ausgestaltung ist dazu vorgesehen, eine mit vorteilhaften Eigenschaften versehene Fassade des Gebäudes zu bilden.

Ein solches Gebäude weist bevorzugt eine Vielzahl von erfindungsgemäßen Fassadenelementen auf, die in der Weise an einer Außenseite des Gebäudes angeordnet sind, dass die Bioreaktoren der Fassadenelemente zumindest einen Teil einer Außenschale des Gebäudes bilden, wobei diese Außenschale in einem Abstand zu einer Gebäudehülle angeordnet ist, so dass zwischen der Gebäudehülle und der Außenschale ein Zwischenraum besteht, wobei die Außenschale insgesamt bevorzugt parallel zur Gebäudehülle verläuft. Die Gebäudehülle kann z.B. eine Außenwand oder ein Abschnitt einer Außenwand oder ein Dach oder ein Abschnitt eines Daches des Gebäudes sein. Eine derartige Ausgestaltung kann zum Beispiel verwirklicht werden, indem eine Fassadenkonstruktion mit der oben beschriebenen Ausgestaltung in der Weise an einer Gebäudehülle befestigt wird, dass eine Außenschale des Gebäudes durch die Fassadenkonstruktion gebildet wird und die die Außenschale bildenden Komponenten der Fassadenkonstruktion in einem Abstand zur Gebäudehülle angeordnet sind, so dass zwischen der Gebäudehülle und der Außenschale ein Zwischenraum besteht. Mit anderen Worten hat die Fassade in diesen Fällen den grundsätzlichen Aufbau einer üblichen zweischaligen Fassade. Die Gebäudehülle kann vertikal, horizontal, geneigt, gekrümmt oder anderweitig geformt sein. Die Fassadenelemente können im Zuge des Neubaus eines Gebäudes oder im nachhinein an bestehenden Gebäuden installiert werden.

Gemäß dieser Ausführungsform mit zweischaliger Anordnung kann der Zwischenraum, der zwischen der eigentlichen Gebäudehülle und der Außenschale bzw. Außenhaut des Gebäudes besteht, die teilweise oder vollständig durch die Bioreaktoren der Fassadenelemente gebildet wird, in vorteilhafter Weise als Nutz- und Erschließungsraum dienen. Die plattenförmigen Photobioreaktoren können hier bei den über Erdgeschoss gelegenen Nutz- oder Erschließungsräumen als Absturzsicherung oder Geländer dienen. Der Zwischenraum kann dabei klimatische Bedingungen aufweisen, die zwischen dem des Makroklimas des Außenraums und dem Miroklima des Gebäudeinneren liegen. Die Bioreaktoren von zumindest einem Teil der Fassadenelemente sind dabei optional bevorzugt in der beschriebenen Weise mit einer Verstelleinrichtung ausgestattet und so ausgestaltet, dass sie jeweils zumindest um eine horizontale und/oder dazu senkrechte Achse geschwenkt werden können, d.h. im Fall von z.B. einer senkrechten Außenwand um eine horizontale und/oder eine vertikale Achse. In diesem Anwendungsfall dienen die Bioreaktoren neben der Produktion von Biomasse und Wärme dem Witterungsschutz und dem Sicht-, Sonnen- und Blendschutz, um den Tageslicht- und Energieeintrag in den Zwischenraum gezielt zu beeinflussen. Durch die Steuerung der Organismendichte in den Bioreaktoren kann die Licht- und Energiedurchlässigkeit gesteuert werden. Zusätzlich dienen die Bioreaktoren dem Schallschutz, indem sie Außenlärm absorbieren. Durch die innere Struktur der Bioreaktoren und ihre Stellung kann eine gezielte Lichtlenkung das Gebäudeinnere erzielt werden. Ist eine Verstelleinrichtung vorgesehen, ermöglichen sie als Lüftungselemente das kontrollierten Zu- und Abführen von Luft in den Zwischenraum. Die Bioreaktoren geben in vorteilhafter Weise Wärme an den Zwischenraum ab und dienen so in den Übergangsjahreszeiten und im Winter auch als "aktive Wärmedämmung", um die Wärmeverluste des Zwischenraums zum Außenklima zu reduzieren. Wenn die Bioreaktoren so ausgerichtet sind, dass eine geschlossene Außenschale bzw. Außenhaut gebildet wird, kann der Zwischenraum als thermischer Puffer dazu beitragen, die Wärmeverluste des Gebäudes zu reduzieren. Dagegen kann bei einer Ausrichtung von zumindest einem Teil der Bioreaktoren mit entsprechenden Verstelleinrichtungen in der Weise, dass die Außenschale geöffnet ist, vor allem im Sommer erwärmte Luft aus dem Zwischenraum abgezogen werden, um eine natürliche Kühlung des Gebäudes zu unterstützen.

Gemäß der Ausführungsform mit zweischaliger Anordnung können die Bioreaktoren alternativ als Außenschale einer hinterlüfteten Außenwandkonstruktion verwendet werden. In diesem Fall weist die Außenwand in der Regel zur Luftschicht hin eine opake oder lichtdurchlässige Dämmebene aus. Die Dämmebene kann auf der Außenseite dunkel- oder hellkaschiert sein, um die Absorption von Sonnenenergie zu steuern, die durch die Bioreaktoren gelangt. In die Außenwandkonstruktion können Fensteröffnungen integriert sein, die zur Belichtung und Belüftung des Innenraumes dienen. Die Photobioreaktoren, die vor diesen Fenster- und Türöffnungen gelegen sind, können hierbei als Absturzsicherung und/oder Vorrichtung zur Lichtlenkung dienen. Die Bioreaktoren von zumindest einem Teil der Fassadenelemente sind dabei optional bevorzugt in der beschriebenen Weise mit einer Verstelleinrichtung ausgestattet und so ausgestaltet, dass sie jeweils zumindest um eine horizontale und/oder dazu senkrechte Achse geschwenkt werden können, d.h. im Fall von z.B. einer senkrechten Außenwand um eine horizontale und/oder eine vertikale Achse. In diesem Anwendungsfall dienen die Bioreaktoren neben der Produktion von Biomasse und Wärme dem Schlagregenschutz der Außenwandkonstruktion. Zusätzlich ermöglichen sie die Verbesserung des Wärmeschutzes und Schallschutzes der Außenwandkonstruktion. Bei einer geschlossenen Stellung der Bioreaktoren, d.h. bei geschlossener Außenschale, führt die Abgabe von Wärme an die Luftschicht und der Energieeintrag durch die Bioreaktoren zu einer Erwärmung der Luftschicht, die die Wärmedämmeigenschaften der Wand verbessern. Im Sommer kann bei Vorsehen von entsprechenden Verstelleinrichtungen durch gezieltes Öffnen im unteren und oberen Bereich der Fassade eine Kamineffekt erzielt werden, der die erwärmten Luftmassen abführt und so zu einer Kühlung der Außenwand beitragen kann. Die abgeführte Wärme kann über Wärmetauscher auf andere Weise dem Gebäude wieder zugeführt werden.

Gemäß der Ausführungsform mit zweischaliger Anordnung können die Bioreaktoren ferner als äußere Verkleidung eines Fassadenelementes verwendet werden, das neben den Bioreaktoren in der oben beschriebenen Weise einen Gebäudehüllenabschnitt aufweist. Mit anderen Worten kann ein solches Fassadenelement einen fertigen Abschnitt einer Außenwand oder eines Daches enthalten, so dass eine Außenwand oder ein Dach durch Zusammensetzen solcher Fassadenelemente aufgebaut werden kann. Derartige Fassadenelemente können im Werk vorgefertigt oder auf der Baustelle montiert werden. Die Gebäudehüllenabschnitte von solchen Fassadenelementen können dabei lichtdurchlässige Bauteile, wie beispielsweise Verglasungen, Kastenfenster oder lichtdurchlässige Wärmedämmungen, oder opake, beispielsweise gedämmte Bauteile sein. Auch in diesem Fall befindet sich zwischen der einen Teil einer Außenschale bzw. Außenhaut bildenden äußeren Verkleidung und dem einen Teil einer Innenschale bildenden Gebäudehüllenabschnitt jedes Fassadenelements eine Luftschicht bzw. ein Zwischenraum, dessen Austausch mit dem Außenraum oder Gebäudeinneren pro Fassadenelement gesteuert werden kann. Die Bioreaktoren von zumindest einem Teil der Fassadenelemente sind dabei optional bevorzugt in der beschriebenen Weise mit einer Verstelleinrichtung ausgestattet und so ausgestaltet, dass sie jeweils zumindest um eine horizontale und/oder dazu senkrechte Achse geschwenkt werden können, d.h. im Fall von z.B. einer senkrechten Außenwand um eine horizontale und/oder eine vertikale Achse, und/oder dass sie jeweils entlang einer dieser Achsen oder entlang beider Achsen verschoben werden können. In diesem Anwendungsfall dienen die Bioreaktoren neben der Produktion von Biomasse und Wärme dem Schlagregenschutz der Gebäudehülle, dem Sicht-, Sonnen- und Blendschutz, um den Tageslicht- und Energieeintrag in den Zwischenraum zu beeinflussen, und bei Bedarf der Absturzsicherung über Erdgeschoss gelegener Nutzungsebenen. Durch die Steuerung der Organismendichte in den Bioreaktoren kann die Licht- und Energiedurchlässigkeit gesteuert werden. Zusätzlich ermöglichen sie die Verbesserung des Wärmschutzes und Schallschutzes der Gebäudehülle. Bei Vorsehen von entsprechenden Verstelleinrichtungen kann durch Schließen und Öffnen der Bioreaktoren die Luftschicht- bzw. der Zwischenraum kontrolliert be- und entlüftet werden. Bei geschlossenen Bioreaktoren trägt die Luftschicht bzw. der Zwischenraum zur Verbesserung des Wärmeschutzes bei. Bei geöffneten Bioreaktoren können solare Energiegewinne in dem Zwischenraum abgeführt werden. Zusätzlich kann über den Zwischenraum und Lüftungselemente in der Gebäudehülle das Gebäudeinnere be- und entlüftet werden.

Es ist weiterhin bevorzugt, wenn bei dem Gebäude in einem Gebäudehüllenabschnitt, wie zum Beispiel einer Außenwand oder einem Dach des Gebäudes, mindestens ein Fenster, eine Tür oder ein verglaster Abschnitt angeordnet ist und mindestens ein erfindungsgemäßes Fassadenelement an dem Gebäudehüllenabschnitt befestigt und in der Weise in Bezug auf das Fenster, die Tür bzw. den verglasten Abschnitt angeordnet ist und/oder in der Position verfahren werden kann, dass es als variabel einstellbarer Sonnenschutz verwendet werden kann. Wie oben beschrieben wurde kann die Einstellung des Sonnenschutzes durch Veränderung der Winkelstellung, der Position (jeweils bei Vorhandensein einer Verstelleinrichtung) und/oder der Transmissionseigenschaften bewirkt werden. Befinden sich Photobioreaktoren vor lichtdurchlässigen Bauteilen wie Fenster- und Türelemente, können die Photobioreaktoren in vorteilhafter Weise auch als Absturzsicherung oder Geländer dienen.

Gemäß einer solchen Ausführungsform können die Bioreaktoren als außenliegende Komponenten an Gebäudehüllen verwendet werden, die teilweise oder vollständig verglast sein können und auch Dächer und ähnlich Gebäudestrukturen umfassen können. An Außenwänden können die Bioreaktoren, wenn sie länglich ausgebildet sind, sowohl liegend als auch stehende angeordnet werden, wobei in letzterem Fall bevorzugt geschosshohe Bioreaktoren verwendet werden. Die Bioreaktoren sind dabei optional bevorzugt in der beschriebenen Weise mit einer Verstelleinrichtung ausgestattet und so ausgestaltet, dass ihre Ausrichtung und Position in Bezug auf die Gebäudehülle verändert werden kann. So können im Fall einer senkrechten Außenwand liegende Bioreaktoren um eine horizontale Achse geschwenkt und/oder um eine vertikale oder in Bezug auf die Außenwand schräge Achse verschoben werden und stehende Bioreaktoren um eine vertikale Achse geschenkt und/oder um eine horizontale oder in Bezug auf die Außenwand schräge Achse verschoben werden. Im Fall eines Flachdaches ist es bevorzugt, dass die Bioreaktoren um eine horizontale und oder vertikale Achse geschwenkt und/oder um eine horizontale oder in Bezug auf das Dach schräge Achse verschoben werden können. In diesen Anwendungsfällen können die Bioreaktoren neben der Produktion von Biomasse und Wärme dem Sonnen- und Blendschutz dienen, um den Tageslicht- und Energieeintrag in das Gebäudeinnere zu beeinflussen. Ferner kann bei Vorsehen von entsprechenden Verstelleinrichtungen durch Verstellen der Neigung des Elementes zur einfallenden Sonne gezielt Licht in das Gebäudeinnere gelenkt werden. Durch die Steuerung der Organismendichte in den Bioreaktoren kann die Licht- und Energiedurchlässigkeit gesteuert werden.

Ein erfindungsgemäßes Gebäude weist bevorzugt eine Wärmetransportvorrichtung auf, die an den Anschluss von mindestens einem erfindungsgemäßen Fassadenelement angeschlossen ist, das zum Beispiel einen Teil der Fassadenkonstruktion bildet. Auf diese Weise ist es möglich, dass Wärme zwischen den Bioreaktor der jeweiligen Fassadenelemente und dem Gebäude ausgetauscht wird. Da Wärme in den Bioreaktoren tendenziell verstärkt im Sommer erzeugt wird, während für einen optimalen Betrieb den Bioreaktoren im Winter tendenziell eher Wärme zugeführt werden muss, ist es vorteilhaft, wenn das Gebäude eine Wärmespeicherungseinrichtung aufweist, die mit der Wärmetransportvorrichtung funktional in der Weise gekoppelt ist, dass Wärme aus den Bioreaktoren von Fassadenelementen, an die die Wärmetransportvorrichtung angeschlossen ist, in der Wärmespeicherungseinrichtung gespeichert werden kann und die Wärmespeicherungseinrichtung Wärme an die Bioreaktoren abgeben kann. Geeignete Wärmespeicherungseinrichtungen sind in vielen modernen energieeffzienten Gebäuden bereits vorhanden. Beispielsweise können Erdsolespeicher, die sowohl als Energiespeicher, als auch als Erdwärmequelle genutzt werden können, oder thermoaktive Bauelemente in Gebäuden, wie zum Beispiel Betonkernaktivierung oder Latentspeicher mit Phasenwechselmaterialien, die beispielsweise unterhalb des Gebäudes angeordnet werden können, Verwendung finden.

Ein bevorzugter Bioreaktor zur Verwendung in erfindungsgemäßen Fassadenelementen zeichnet sich unter anderem durch eine hohe Lichtausnutzung durch die phototrophen Organismen für eine hohe Biomasseproduktion aus und ist in der europäischen Patentanmeldung Nr. 09154581.4 beschrieben. Bei der Lichtausnutzung besteht im allgemeinen das Problem, dass die Lichtintensität aufgrund der Lichtabsorption durch die Organismen und in dem Kulturmedium und durch Abschattungseffekte mit zunehmender Eindringtiefe in das Kulturmedium stark abnimmt, so dass bei hoher Dichte der Organismen eine ausreichende Lichtzufuhr lediglich in einer gewissen Schicht in der Nähe der belichteten Gehäusebereiche gewährleistet ist. Darüber hinaus ist auch zu beachten, dass phototrophe Organismen einfallendes Licht nicht kontinuierlich verarbeiten können, sondern theoretisch sogar nur während eines Drittels der Zeit Licht aufnehmen und für die Energiegewinnung nutzen können. Aus diesem Grund wird eine optimale Lichtausnutzung und Photosyntheseaktivität der phototrophen Organismen erreicht, wenn die Organismen im statistischen Mittel einen Hell-Dunkel-Zyklus durchlaufen, so dass sie während eines ersten Zeitraums Licht relativ hoher Intensität aufnehmen und anschließend für einen zweiten Zeitraum in relative Dunkelheit zurückkehren. Auf diese Weise können zum einen mehr Organismen an der phototrophen Aktivität teilnehmen, und zum anderen sind die Organismen nur für kurze Zeit maximaler Lichtintensität ausgesetzt, die bei Starklicht sogar zu einer Schädigung der Organismen (sog. Photoinhibition) führen kann.

Um vor diesem Hintergrund eine hohe Lichtausnutzung zu gewährleisten kann in vorteilhafter Weise ein Bioreaktor mit einem quaderförmigen Gehäuse zum Einsatz kommen, in dessen Reaktorraum mehrere Zwischenwände oder Leitplatten angeordnet sind, die bevorzugt ebenfalls aus einem lichtdurchlässigen Material, wie zum Beispiel einem der oben in Zusammenhang mit dem Gehäuse genannten Materialien, ausgebildet sind. Diese Zwischenwände verlaufen parallel zu den kleineren Seitenwänden und parallel zueinander, d.h. sie erstrecken sich entlang der Länge des Reaktorraums und des Gehäuses. Jede Zwischenwand erstreckt sich über die gesamte Dicke des Reaktorraums, so dass sie die beiden größeren Seitenwände miteinander verbindet. Die Zwischenwände sind in der Nähe einer ersten der beiden Stirnwände und, zum Beispiel bevorzugt 2 bis 20 cm und mehr bevorzugt 6 bis 10 cm, beabstandet von dieser ersten Stirnwand angeordnet oder weisen eine Öffnung oder einen Spalt im Bereich der ersten Stirnwand auf. Dabei sind die Zwischenwände bevorzugt alle gleich weit von der ersten Stirnwand beabstandet.

Die Zwischenwände erstrecken sich über einen Teil der Länge des Reaktorraums. Auf diese Weise wird in der Nähe der ersten Stirnwand ein Strömungsleitabschnitt des Reaktorraums mit einer Vielzahl von Strömungsleitkanälen gebildet, von denen sich jeder zwischen zwei benachbarten Zwischenwänden oder zwischen einer der kleineren Seitenwände und einer benachbarten Zwischenwand befindet. Zwischen den der ersten Stirnwand abgewandten Enden der Zwischenwände und der zweiten der beiden Stirnwände ist ein freier bzw. im wesentlichen freier Durchmischungsabschnitt des Reaktorinnenraums vorgesehen, in dem sich keine Zwischenwände befinden. Die Zwischenwände haben bevorzugt identische Längen. Ferner ist es bevorzugt, dass die Zwischenwände gleichmäßig voneinander und von den kleineren Seitenwänden beabstandet sind. Dabei sind die Zwischenwände so angeordnet und dimensioniert, dass sich der Durchmischungsabschnitt des Reaktorraums über mindestens 50%, bevorzugt mindestens 60% und am meisten bevorzugt mindestens 80 % der Länge des Reaktorraums erstreckt und dass die Strömungsleitkanäle eine Breite, d.h. einen Abstand zwischen benachbarten Zwischenwänden bzw. zwischen einer kleinen Seitenwand und der benachbarten Zwischenwand, von mindestens 5 cm aufweisen. Ferner ist es bevorzugt, dass sich die Strömungsleitkanäle über mindestens 10% der Länge des Reaktorraums erstrecken. Es ist ferner bevorzugt, dass die Länge der Strömungsleitkanäle 10 bis 200 cm und mehr bevorzugt 60 bis 100 cm beträgt. In einer vorteilhaften Ausgestaltung sind mindestens fünf und bevorzugt sieben bis neun Strömungsleitkanäle vorgesehen. Die Zwischenwände und Flanken der Strömungsleitkanäle können bei Bedarf durch entsprechende Ausbildung dem Sonnenschutz, Blendschutz bzw. der Tageslichtlenkung dienen.

In der Nähe der ersten Stirnwand ist eine Gaszufuhreinrichtung zum Einbringen von Gas in jeden zweiten der Strömungsleitkanäle angeordnet. Im Betrieb wird mit Hilfe der Gaszufuhreinrichtung gleichzeitig Gas in das der ersten Stirnwand zugewandte Ende jedes zweiten der Strömungsleitkanäle eingedüst. Um eine Bewegung des Gases entlang der Strömungsleitkanäle zu erhalten, wird der Bioreaktor dabei so angeordnet, dass die erste Stirnwand tiefer als die zweite Stirnwand liegt und die erste Stirnwand somit im Anwendungsfall die Unterseite des Bioreaktors bildet. Aufgrund der Breite der Strömungsleitkanäle von mindestens 5 cm kann sich in diesen bei hinreichender Begasungsrate und -art eine durch den Gaseintrag induzierte Flüssigkeitsströmung hoher Geschwindigkeit ausbilden. Das Gas steigt in jedem zweiten Strömungsleitkanal nach oben und erzeugt durch den Auftrieb einen Flüssigkeitsstrom nach oben (Airlift). Dabei reißt zudem das Gas Flüssigkeit vom unteren Ende des Reaktorraums mit, und die Flüssigkeitssäulen in den nicht begasten Strömungsleitkanälen drücken aufgrund ihrer größeren Masse die Flüssigkeit in den begasten Strömungsleitkanälen nach oben. In den nicht begasten Strömungsleitkanälen bewirkt der Sog der in den begasten Strömungsleitkanälen aufsteigenden Flüssigkeit, dass sich in den nicht begasten Strömungsleitkanälen eine Flüssigkeitsströmung nach unten ausbildet. Durch diese wird Flüssigkeit aus dem Oberteil des Bioreaktors bis auf dessen Boden in der Nähe der ersten Stirnwand transportiert. In der Realisierung dieses Konvektionsprinzips wird demnach alternierend jeder zweite Strömungsleitkanal begast, unabhängig von der Breite des Bioreaktors und der Anzahl der Strömungsleitkanäle.

Erreicht das in die Strömungsleitkanäle eingedüste Gas das obere Ende der Zwischenwände, und damit das Ende des Strömungsleitabschnitts des Reaktorraums, so steigt das Gas weiter auf und erzeugen zusammen mit den ab- und aufsteigenden Flüssigkeitsströmungen eine stark turbulente dreidimensionale Durchmischung in dem Durchmischungsabschnitt des Reaktorraums und ggf. auch in den Strömungsleitkanälen.

Durch den gewählten Aufbau ist es überraschenderweise möglich, auch außerhalb von Strömungsleitkanälen überall in einem ausgedehnten Durchmischungsbereich, der mehr als die Hälfte der Länge des Reaktorraums einnimmt und aus diesem Grunde für die Lichtausnutzung und Kultivierung bestimmend ist, eine sehr starke turbulente Durchmischung zu erzielen, die zu sehr hohen Frequenzen des Hell-Dunkel-Zyklus der Organismen mit einer Zykluszeit von bevorzugt einigen Millisekunden führt. Dies lässt sich zudem auch mit einer geringen Dicke des Bioreaktors bzw. Reaktorraums von bevorzugt 1,5 bis 5 cm erzielen, so dass in vorteilhafter Weise ein großes Verhältnis von beleuchtbarer Gehäuseseitenfläche zu Reaktorraumvolumen realisiert werden kann. Ferner ist es durch die erzielbare starke Turbulenz und den damit verbundenen schnellen periodischen Wechsel der Organismen zwischen der lichtzugewandten beleuchteten Seite des Bioreaktors und den dunklen Bereichen in der Mitte des Reaktorraums - bzw. in der Nähe der lichtabgewandten Seitenfläche, falls der Bioreaktor nur einseitig belichtet wird - möglich, hohe Zelldichten zu kultivieren. Zudem bewirkt die hohe Turbulenz, dass die Organismen so stark bewegt werden, dass sie immer optimal mit den im Kulturmedium gelösten Nährstoffen und mit Gasen versorgt werden und dass sie sich nicht absetzen können. Die starke Turbulenz verhindert auf diese Weise zusammen mit aufsteigenden Gasblasen auch eine Besiedelung der Reaktoroberflächen (Biofilmbildung bzw. Biofouling), die eine Verminderung des Lichteintritts in den Reaktorraum zur Folge hätte.

Beispielsweise ist dieser Bioreaktor in einer bevorzugten Ausführungsform durch geeignete Wahl der Breite der Strömungsleitkanäle und der übrigen Dimensionen des Bioreaktors so ausgestaltet, dass durch das beschriebene Einbringen von Gas und das beschriebene Aufsteigen von Gasblasen in den Strömungsleitkanälen im gesamten Durchmischungsbereich oder im wesentlichen im gesamten Durchmischungsbereich aufgrund der erzeugten Turbulenz für die Organismen ein Wechsel zwischen hell und dunkel mit einzelnen Dunkelphasen von 10 ms oder weniger bewirkt werden kann und/oder ein turbulenter Stoffaustauschkoeffizient von mindestens 0,016 m²/s erzielt werden kann. Letzteres führt in vorteilhafter Weise dazu, dass räumliche Konzentrationsunterschiede im Reaktorraum weniger als 2% betragen.

Der Reaktorraum dieses Bioreaktors ist demnach im Vergleich zu Bioreaktoren des Standes der Technik weitergehend für die Kultivierung von phototrophen Organismen optimiert, wobei sich die Optimierung darauf bezieht, die Organismen mit Licht zu versorgen, die Organismen mit im Kulturmedium gelösten Nährstoffen (Stickstoff, Phosphor und Spurenelemente) und Gasen zu versorgen, zu verhindern, dass sich die Organismen absetzen bzw. sedimentieren, und zu verhindern, dass sich Organismen an den Reaktorwänden anheften, wodurch stets ein ungehinderter Lichteintritt in den Reaktorraum gewährleistet ist.

Ferner lässt sich ein im Vergleich zu anderen Reaktortypen günstigeres Verhältnis von Oberfläche und Volumen verwirklichen, d.h. die plattenförmigen Bioreaktoren können sehr dünn und deshalb für geringe Volumina an Kulturmedium ausgelegt werden. So weist der Reaktorraum in einer bevorzugten Ausgestaltung des Bioreaktors eine Dicke von 1,5 bis 5 cm auf, mehr bevorzugt von 2,0 bis 3,0 cm. Die Länge des Reaktorraums beträgt bevorzugt 100 bis 600 cm, mehr bevorzugt 300 bis 400 cm, und die Breite des Reaktorraums beträgt bevorzugt 30 bis 400 cm, mehr bevorzugt 100 bis 200 cm. In jedem Fall ist es vorteilhaft, wenn die Länge des Reaktorraums größer als seine Breite ist.

Durch das günstige Verhältnis von Oberfläche und Volumen ist es in vorteilhafter Weise möglich, den Einsatz an Ressourcen zur Kultivierung (u.a. Wasser, Nährstoffe, Gas und Energie) zu verringern. Wie auch bei anderen Reaktortypen werden im Betrieb bei dem Wunsch einer niedrigen Transmission bevorzugt Zelldichten im Reaktorraum eingestellt, die eine optische Dichte von 7 oder ungefähr 7 bewirken, was hier einem Hell-Dunkel-Aufenthaltszeitverhältnis von etwa 1:2 bis 1:3 entspricht. Diese Einstellung kann über die Zelldichte als Solltrockenmasse in g/l erfolgen. Diese beträgt beispielsweise bei einer Dicke des Bioreaktors von 5 cm bevorzugt 0,9 g/l, bei einer Dicke von 3 cm bevorzugt 1,5 g/l und bei einer Dicke von 1,5 cm bevorzugt 3 g/l. Bis zu diesen optisch begründeten Solltrockenmassen findet keine Unterversorgung der Organismen mit Licht statt, so dass alle Organismen gleichmäßig photosynthetisch aktiv gehalten werden können.

Im Rahmen erfindungsgemäßer Fassadenelemente eignen sich diese Bioreaktoren besonders gut, wenn die Fassadenelemente eine Fläche von mehr als einem Quadratmeter bereitstellen sollen. Dabei ist die Möglichkeit von besonderem Vorteil, den Bioreaktor mit geringem Energie- und Ressourceneinsatz zu betreiben und eine hohe Produktion von Biomasse pro Reaktorfläche zu erzielen.

In einer vorteilhaften Ausgestaltung dieses Bioreaktors beträgt die Breite der Strömungsleitkanäle mindestens 10 cm und bevorzugt 10 bis 50 cm. In einer weiteren vorteilhaften Ausgestaltung beträgt die Breite der Strömungsleitkanäle mindestens 15 cm und bevorzugt 15 bis 20 cm.

In einer bevorzugten Ausführungsform dieses Bioreaktors sind in den Zwischenwänden entlang ihrer Länge jeweils eine Anzahl voneinander beabstandeter Öffnungen bzw. Durchgangsbohrungen vorgesehen, durch die ein Flüssigkeitsaustausch zwischen benachbarten Strömungsleitkanälen stattfinden kann. Durch diese Ausgestaltung wird bewirkt, dass das in jedem zweiten Strömungsleitkanal aufsteigende Gas bei der hydrostatischen Erzeugung einer Flüssigkeitsströmung nach oben nicht nur Flüssigkeit vom unteren Ende des Reaktorraums, d.h. aus der Nähe der ersten Stirnwand, mitreißt, sondern auch zusätzlich über die Öffnungen aus den angrenzenden, nicht begasten Strömungsleitkanälen. Dadurch wird erreicht, dass die Gasblasen in den begasten Strömungskanälen über den gesamten Strömungskanal ungehindert aufsteigen können, hohe Geschwindigkeiten erreichen und somit hohe Strömungsgeschwindigkeiten erzielt werden können. Darüber hinaus entstehen durch das seitliche Einströmen von Flüssigkeit in die begasten Strömungskanäle in den Strömungsleitkanälen Verwirbelungen, so dass nicht nur im Durchmischungsbereich, sondern auch im Bereich der Strömungsleitkanäle turbulente Durchmischungen hoher Geschwindigkeit stattfinden können. Es ist besonders bevorzugt, wenn die Öffnungen bzw. Durchgangsbohrungen in Bereichen der Zwischenwände vorgesehen sind, die an eine der großen Seitenwände des Gehäuses angrenzen, und zwar an die große Seitenwand, die im Betrieb nicht dazu vorgesehen ist, von Licht durchstrahlt zu werden, d.h. die große Seitenwand, die bei einer geneigten Anordnung des Bioreaktors der sonnenabgewandten Seite entspricht. Dadurch wird verhindert, dass Teile der aufsteigenden großen Gasblasen ganz oder teilweise in diejenigen Strömungsleitkanäle gelangen, in denen die Flüssigkeit nach unten strömt, dort aufsteigen und so die Flüssigkeitsströmung abbremsen. Die Öffnungen bzw. Durchgangsbohrungen können in einer vorteilhaften Ausgestaltung Längen in Richtung des Verlaufs der Strömungsleitkanäle von 1 bis 30 cm und mehr bevorzugt 3 bis 5 cm und Abmessungen senkrecht zum Verlauf der Strömungsleitkanäle von 10 bis 90% und mehr bevorzugt von 50% oder etwa 50% der Höhe der Zwischenwände aufweisen.

Es ist bevorzugt, dass die Gaszufuhreinrichtung ein Rohr oder mehrere Rohre aufweist, das bzw. die in dem Reaktorraum zwischen der ersten Stirnwand und den dieser zugewandten Enden der Zwischenwände verläuft bzw. verlaufen und Bohrungen oder Schlitze aufweist bzw. aufweisen, durch die Gas in die jeweiligen zu begasenden Strömungsleitkanäle abgegeben wird. Dabei ist es ferner bevorzugt, dass das Rohr bzw. die Rohre parallel zu der ersten Stirnwand verläuft bzw. verlaufen. Die Dicke des Rohrs bzw. der Rohre sollte bevorzugt nicht mehr als ein Drittel der Dicke des Reaktorraums betragen, um die Strömung nicht zu stark zu behindern. Aus demselben Grunde ist es vorteilhaft, wenn das Rohr bzw. die Rohre in einem gewissen Abstand zu den der ersten Stirnwand zugewandten Enden der Zwischenwände angeordnet sind. Zum Beispiel ist es bevorzugt, dass das Rohr bzw. die Rohre in einem Abstand von 5 bis 150 mm und mehr bevorzugt von 30 bis 50 mm von der ersten Stirnwand angeordnet sind. Ferner ist es bevorzugt, dass das Rohr bzw. die Rohre in einem Abstand von 5 bis 150 mm und mehr bevorzugt von 30 bis 50 mm von den der ersten Stirnwand zugewandten Enden der Zwischenwände angeordnet sind. Das Rohr sollte ferner auch deshalb einen möglichst geringen Innendurchmesser aufweisen, weil bei einer stossweisen Begasung Flüssigkeit ins Rohr eintritt und mit dem nächsten Stoß zunächst erst wieder ausgeblasen werden muß. Bei dünnen Rohren kommt es daher zu weniger Verzögerungszeit. Beispielsweise beträgt der Innendurchmesser des Begasungsrohres für eine Reaktorbreite von 1 m bevorzugt 5 mm.

Ein weiterer vorteilhafter Gaseintrag kann über sogenannte Rückschlagventile erfolgen, die jeweils einzeln am unteren Ende und mittig eines jeden Strömungskanals so angebracht sind, dass der Ausgang des Ventils frei in den Reaktorinnenraum ragt. Solch eine Anordnung ermöglicht den Gaseintrag in jeden einzelnen Strömungskanal unabhängig voneinander durchzuführen. Im einfachsten Fall können so zeitlich versetzt nebeneinander liegende Kanäle begast bzw. nicht begast werden. Über solch eine Gaszuführung wird erreicht, dass die Richtung der Wasserströmung in den Kanälen mit dem Gaseintrag periodisch wechselt, was vorteilhaft in Bezug auf die Antifoulingwirkung der Strömung ist.

In Zusammenhang mit einer solchen Gaszufuhreinrichtung ist es ferner vorteilhaft, wenn die Gaszufuhreinrichtung angepasst ist, um, bevorzugt automatisch, beispielsweise mit Hilfe einer geeigneten Steuereinrichtung, die Teil des Bioreaktors sein kann, bei mit flüssigem Kulturmedium und phototrophen Organismen befülltem Reaktorraum Gas in Form einzelner, zusammenhängender Gasblasen in die jeweiligen Strömungsleitkanäle einzubringen. Die Gasblasen weisen dabei einen großen Durchmesser (d.h. der maximale Durchmesser einer gegebenen Gasblase) von mindestens 3 cm, bevorzugt von 3 bis 50 cm und mehr bevorzugt von 10 bis 15 cm auf. Durch den Aufstieg derartiger einzelner, großer Gasblasen in den Strömungsleitkanälen wird eine hohe Geschwindigkeit des Gasaufstiegs und infolgedessen eine hohe Sogwirkung erreicht, was für die im Durchmischungsabschnitt zu erzielende starke Turbulenz günstige hohe Strömungsgeschwindigkeiten mit sich bringt, die in den Strömungsleitkanälen mindestens so groß wie die Gasblasengeschwindigkeit ist. Es ist besonders bevorzugt, wenn die Gasblasen einen großen Durchmesser aufweisen, der der Breite des jeweiligen Strömungsleitkanals entspricht oder größer ist. Eine solche Blase wirkt wie ein Saugheber bzw. Kolben und entwickelt somit die größte Sogwirkung. Die Abmessungen der Schlitze bzw. die Größe der Bohrungen in dem Rohr bzw. den Rohren richtet sich im allgemeinen nach der Menge des einzublasenden Gases bzw. dem Druck mit dem es eingeblasen wird und sollen bevorzugt groß genug sein, damit in einem Zeitraum von 100 bis 1000 ms die oben genannten Blasengrößen erreicht werden. Bevorzugt haben die Bohrungen einen Durchmesser von 0,5 bis 4 mm und mehr bevorzugt 1 bis 2 mm, und die Schlitze haben bevorzugt eine Breite von 0,2 bis 1 mm und mehr bevorzugt von 0,4 bis 0,6 mm. Es ist dabei ferner bevorzugt, dass die Gaszufuhreinrichtung angepasst ist, um das Gas so einzubringen, dass die Gasblasengeschwindigkeit in den Strömungsleitkanälen mindestens 0,5 m/s beträgt. Die Größe und Geschwindigkeit der Gasblasen wird allgemein so gewählt, dass die oben angegebenen vorteilhaften Strömungs- und Turbulenzcharakteristika entstehen. Insbesondere ist es bevorzugt, wenn die Größe und die Geschwindigkeit der Gasblasen in der Weise auf die Ausgestaltung des Bioreaktors abgestimmt sind, dass in der gesamten oder im wesentlichen in der gesamten Flüssigkeit im Reaktorraum überkritische Reynoldszahlen vorliegen, so dass in der gesamten oder im wesentlichen in der gesamten Flüssigkeit turbulente Strömungsbedingungen herrschen.

Um den Antrieb mit möglichst wenig Gas zu erreichen, ist es ferner bevorzugt, wenn die Gaszufuhreinrichtung angepasst ist, um das Gas, bevorzugt automatisch, beispielsweise mit Hilfe einer geeigneten Steuereinrichtung, die Teil des Bioreaktors sein kann, in der Weise stoßweise in die entsprechenden Strömungsleitkanäle einzubringen, dass erst dann eine neue Gasblase erzeugt wird, wenn die vorhergehende Gasblase das Kulturmedium in dem Reaktorraum vollständig durchlaufen hat. In einer bevorzugten Ausführungsform beträgt die Impulslänge des stoßweisen Einbringens von Gas 100 bis 1500 ms mit einer Periodizität von 500 bis 10000 ms und mehr bevorzugt 400 bis 600 ms mit einer Periodizität von 1500 bis 4000 ms.

Insgesamt kann im Rahmen der vorliegenden Erfindung zur Bewirkung der Flüssigkeitsströmung in vorteilhafter Weise mit geringen Gasdrücken von bevorzugt 0,1 bis 2,0 bar Überdruck und mehr bevorzugt 0,5 bis 1,0 bar Überdruck gearbeitet werden.

In einer bevorzugten Ausführungsform dieses Bioreaktors sind in dem Durchmischungsabschnitt des Reaktorraums eine Vielzahl voneinander beabstandeter Säulen oder Stäbe angeordnet, die jeweils die beiden großen Seitenwände des Gehäuses miteinander verbinden und bevorzugt jeweils so ausgebildet sind, dass sie sich zumindest in Richtung auf die große Seitenwand, z.B. konisch, verjüngen, die der vorgesehenen Lichteinstrahlung abgewandt ist. Durch diese Ausgestaltung wird in vorteilhafter Weise die mechanische Festigkeit gegenüber Vibrationen und Schwingungen der Gehäusewände und der Zwischenwände erhöht, die im Betrieb aufgrund des Flüssigkeitsdrucks auftreten können. Allgemein dienen derartige Säulen oder Stäbe dazu, die Gehäusekonstruktion mechanisch zu stabilisieren. Diese Stabilisierungsfunktion wird im Strömungsleitabschnitt des Reaktorraums von den Zwischenwänden übernommen. Außerdem wirken die Säulen oder Stäbe wie statische Mischer an denen sich die Flüssigkeitsströmungen brechen, was die turbulente Durchmischung weiter erhöht. Die Säulen bzw. Stäbe können maximale Querschnittsdurchmesser von 1 bis 10 cm und mehr bevorzugt von 3 bis 5 cm aufweisen.

Ein derart aufgebauter Bioreaktor kann in vorteilhafter Weise aus zwei Halbschalen gefertigt werden, die zum Beispiel durch Tiefziehen hergestellt und anschließend miteinander verbunden werden, oder in einem Stück durch Twin Sheet oder beispielsweise Spritzgieß- oder Rotationsverfahren.

Werden mehrere dieser Bioreaktoren als Batch oder als Turbidostat betrieben, ist der Ablaufanschluss bevorzugt am oberen Ende des Bioreaktors angeordnet sein, d.h. an der zweiten Stirnwand vorgesehen sein, und der Ablauf kann bevorzugt durch diesen Ablaufanschluss zusammen mit der Abgabe von Gas aus dem Reaktorraum stattfinden. Nach Trennung des Gas-Wasser-Gemisches kann die Flüssigkeit dann alleine über den hydrostatischen Druck bis zur Pumpe geführt werden.

Sind Diffusoren vorgesehen, so sind sie bevorzugt im Bereich zwischen der ersten Stirnwand und den dieser zugewandten Enden der Zwischenwände im Reaktorraum angeordnet und weisen einen, beispielsweise durch eine der größeren Seitenwände vorgesehenen Zulauf auf, über den die Beaufschlagung mit Gas erfolgt.

Der soeben beschriebene spezielle Bioreaktor kann vorteilhaft im Rahmen der Kultivierung der oben genannten phototrophen Organismen verwendet werden, indem ein flüssiges Kulturmedium und photothrophe Organismen in den Reaktorraum des Bioreaktors eingebracht werden und die gebäudeabgewandte Seite der beiden größeren Seitenwände des Gehäuses des Bioreaktors derart mit Licht bestrahlt wird, dass das Licht in den Reaktorraum gelangt. Gleichzeitig wird in jeden zweiten Strömungsleitkanal mit Hilfe der Gaszufuhreinrichtung Gas eingedüst. Dieses Eindüsen erfolgt in der Weise, dass das Gas, wie oben bereits beschrieben wurde, in Form einzelner Blasen mit einem großen Durchmesser von mindestens 3 cm in den entsprechenden Strömungsleitkanälen aufsteigt. Es hat sich gezeigt, dass es auf diese Weise aufgrund der besonderen Ausgestaltung des Bioreaktors möglich ist, in der oben beschriebenen Weise eine ausreichend starke dreidimensionale Turbulenz mit den bereits angegebenen Eigenschaften nicht nur in den Strömungskanälen, sondern auch im gesamten ausgedehnten Durchmischungsbereich zu erreichen, verbunden mit der oben beschriebenen vorteilhaften Bewegung der phototrophen Organismen.

In einer bevorzugten Ausgestaltung wird das Gas mit Hilfe der Gaszufuhreinrichtung so eingedüst, dass im gesamten Durchmischungsbereich oder im wesentlichen im gesamten Durchmischungsbereich aufgrund der erzeugten Turbulenz ein Wechsel der Organismen zwischen hell und dunkel mit einzelnen Dunkelphasen von 10 ms oder weniger bewirkt wird und/oder ein turbulenter Stoffaustauschkoeffizient von mindestens 0,016 m²/s erzielt wird. Letzteres führt in vorteilhafter Weise dazu, dass räumliche Konzentrationsunterschiede im Reaktorraum weniger als 2% betragen.

Die Größe und Geschwindigkeit der Gasblasen wird so gewählt, dass die gewünschten vorteilhaften Strömungs- und Turbulenzcharakteristika entstehen, bevorzugt mit überkritischen Reynoldszahlen in der gesamten oder im wesentlichen in der gesamten Flüssigkeit im Reaktorraum. Es ist bevorzugt, dass das Gas mit Hilfe der Gaszufuhreinrichtung so eingebracht wird, dass die Geschwindigkeit der das Kulturmedium in den Strömungsleitkanälen durchlaufenden Gasblasen mindestens 0,5 m/sec beträgt.

Es ist ferner bevorzugt, dass das zum Antreiben der Flüssigkeitsströmung verwendete Gas mit einem Volumenstrom pro Fläche der Stirnwand, über die das Gas eingebracht wird, (sog. Filtergeschwindigkeit) von mindestens 0,02 m/sec in die begasten Strömungskanäle eingebracht wird.

Außerdem ist es bevorzugt, dass das Gas in der Weise stoßweise, und bevorzugt periodisch, in die entsprechenden Strömungsleitkanäle eingebracht wird, dass erst dann eine neue Gasblase erzeugt wird, wenn die vorhergehende Gasblase das Kulturmedium in dem Reaktorraum vollständig durchlaufen hat. Auf diese Weise kann die angestrebte stark turbulente Bewegung mit möglichst wenig Gas erreicht werden. Die Länge der Periode, mit der der Gaseintrag erfolgt, richtet sich nach der Zeit, die eine Gasblase braucht, um bis zur Oberkante des Reaktors bzw. bis zur der zweiten Stirnwand des Gehäuses benachbarten Flüssigkeitsoberfläche des Kulturmediums zu gelangen. Die Länge des Gasstoßes richtet sich nach der Größe der dabei zu erzeugenden Gasblase.

Der Aufbau des Bioreaktors sowie die Kreislaufführung als Turbidostat ermöglichen ein hohes Maß an Automatisierung der Prozess- bzw. Anlagenführung. Insbesondere ist es in vorteilhafter Weise möglich, über die Größe und Periodizität des Gaseintrags in den Reaktorraum die Strömungsgeschwindigkeit und dementsprechend die Periodizität des Hell-Dunkel-Zyklus einzustellen bzw. zu variieren. Das Lichtklima im Reaktorraum kann über die Zelldichte sowie über die Variation des Neigungswinkels bzw. die Ausrichtung des Reaktors zur Sonne im Tages- und Jahresverlauf eingestellt werden. Bei einer Kreislaufführung können sämtliche Wasserinhaltsstoffe kontrolliert bzw. variiert werden, und es kann in einfacher Weise eine Ernte der Organismen erfolgen.

Es ist vorteilhaft, wenn das beschriebene Verfahren mit einem Bioreaktor oder mit mehreren, miteinander verbundenen Bioreaktoren durchgeführt wird, wobei Teilströme an Kulturmedium gleichzeitig mit der Zufuhr von neuem oder zurück geführtem Kulturmedium kontinuierlich aus dem Reaktor ausgeschleust werden. Im Verlaufe des Verfahrens werden Nährstoffe, Wäre, Substanzen zur Einstellung des pH-Wertes und Gase in den Reaktorraum bzw. in die Reaktorräume eingebracht und phototrophe Organismen geerntet. Nährstoffe können einzeln oder als Mischung in wässriger Form zudosiert werden, sobald sie unter einen Grenzwert absinken, der in geeigneter Weise messtechnisch überwacht werden kann. Als Nährstoffe können sowohl mineralische als auch organische Dünger verwendet werden. Als organischer Dünger ist Gülle aus der Masttierhaltung vorteilhaft. Wärme kann in vorteilhafter Weise dann über einen Wärmetauscher in das Kulturmedium eingebracht werden, wenn die Temperatur des Kulturmediums im Reaktor 10 °C unterschreitet, und zwar in der Weise, dass die Temperatur auf 12 bis 30 °C gebracht wird. Basen, Säuren oder Puffer werden bevorzugt dann hinzudosiert, wenn ein Grenzwert für pH unterschritten wird, der in geeigneter Weise messtechnisch überwacht werden kann. Die Gase werden in einer vorteilhaften Ausgestaltung über einen statischen Mischer bis zur Sättigung in das Kulturmedium eingebracht. Die phototrophen Organismen werden bevorzugt bei Überschreitung eines Grenzwertes für die Zellzahl bzw. der damit korrelierenden Trübung aus dem Kulturmedium abgetrennt.

Die Erfindung wird im Folgenden anhand von bevorzugten Ausführungsbeispielen unter Bezugnahme auf die beigefügten Zeichnungen beschrieben.
Figur 1 zeigt schematisch ein Gebäude mit einer senkrechten Außenwand, an der als Sonnen- und Blendschutz dienende erfindungsgemäße Fassadenelemente befestigt sind.
Figur 2 zeigt schematisch ein Gebäude mit einem horizontalen Dach, an dem als Sonnen- und Blendschutz dienende erfindungsgemäße Fassadenelemente befestigt sind.
Figur 3 zeigt schematisch ein Gebäude mit einer durch erfindungsgemäße Fassadenelemente gebildeten Außenschale bzw. Außenhaut, die vor der durch eine senkrechte Außenwand gebildeten eigentlichen Gebäudehülle angeordnet ist, so dass zwischen der Außenschale und der Gebäudehülle ein Zwischenraum besteht, der als Nutz- und Erschließungsraum dienen kann.
Figur 4 zeigt schematisch ein Gebäude mit einer hinterlüfteten Außenwandkonstruktion, deren Außenschale durch erfindungsgemäße Fassadenelemente gebildet wird.
Figur 5 zeigt schematisch ein Gebäude mit einer senkrechten Außenwand, die durch erfindungsgemäße Fassadenelemente gebildet wird, die neben einem Bioreaktor einen Gebäudehüllenabschnitt aufweisen.
Figur 6 zeigt schematisch verschiedene Fassadenelemente, in denen die Bioreaktoren für ein Verschwenken um verschiedene Achsen angeordnet sind.

In verschiedenen in den Figuren dargestellten Ausführungsbeispielen sind gleiche oder ähnliche Elemente und Komponenten jeweils durch identische Bezugszeichen gekennzeichnet.

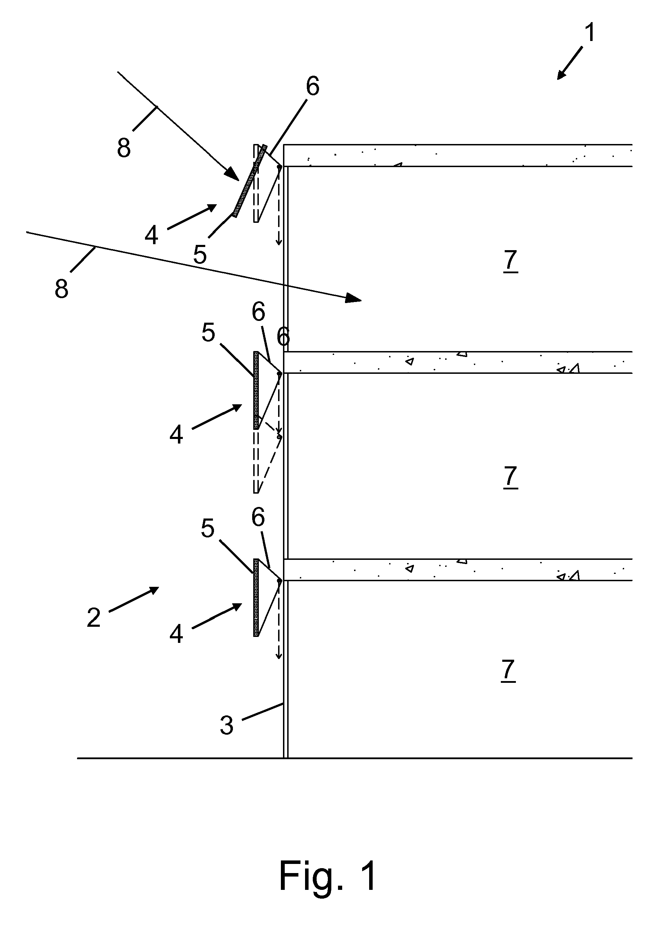

Das in Figur 1 gezeigte Gebäude 1 weist eine Fassade 2 auf, die eine senkrechte Außenwand 3, die einen Abschnitt einer Gebäudehülle bildet, und drei Fassadenelemente 4 umfasst. Jedes Fassadenelement 4 weist einen plattenförmigen Bioreaktor 5 und eine Befestigungseinrichtung 6 in Form eines Trägers auf, der an der Außenwand 3 befestigt ist und die Bioreaktoren 5 von der Außenwand 3 beabstandet hält. Die Befestigung an der Außenwand 3 ist dabei in der Weise ausgestaltet, dass es möglich ist, die Winkelstellung der Bioreaktoren 5 durch Verschwenken um eine horizontale Achse aus der für die unteren beiden Bioreaktoren 5 gezeigten Stellung parallel zur Außenwand 3 in eine für den obersten Bioreaktor 5 gezeigte Stellung geändert werden kann, in der der Bioreaktor 5 in Bezug auf die Außenwand schräg verläuft. Darüber hinaus ist die Befestigung an der Außenwand 3 auch in der Weise ausgestaltet, z.B. durch geeignete Schienen, dass die Position der Bioreaktoren 5 entlang der Außenwand 3 nach oben und unten verändert werden kann, d.h. entlang einer vertikalen Achse. Dies ist durch die gestrichelten Darstellungen der Fassadenelemente 4 und die gestrichelten Pfeile angedeutet. Es kann vorgesehen sein, dass das Verschwenken und die Positionsveränderung manuell vorgenommen werden oder dass entsprechende Motoren vorhanden sind.

Die Fassadenelemente 4 können im Zuge des Neubaus des Gebäudes 1 oder im nachhinein an dem ansonsten bereits bestehenden Gebäude 1 installiert werden.

In den einzelnen Etagen ist jeweils ein Fenster oder ein anderer verglaster Bereich (nicht gezeigt) in der Außenwand 3 ausgebildet, d.h. die Gebäudehülle 3 kann teilweise oder vollständig verglast sein. Die Bioreaktoren 5 der Fassadenelemente 4 sind länglich und rechteckig ausgebildet und haben eine Länge, die so gewählt ist, dass sie sich über die gesamte Länge des Fensters bzw. verglasten Bereichs erstrecken. Somit sind sie in liegender Ausrichtung angeordnet. Es ist nun ersichtlich, dass je nach Sonnenstand und je nach gewünschter Abschattung ein durch die Fassadenelemente 4 bereitgestellter Sonnen- und Blendschutz und der Eintrag von Wärme ins Gebäudeinnere durch Sonneneinstrahlung durch Einstellen der Position, der Winkelstellung und/oder der Transmissionseigenschaften der Bioreaktoren 5 individuell und sehr flexible angepasst werden können. Die Anpassung bewirkt, dass gezielt mehr oder weniger Sonnenstrahlen 8 auf dem Weg ins Gebäudeinnere von den Bioreaktoren 5 absorbiert werden. Dabei werden die Bioreaktoren 5 gleichzeitig zur Energiegewinnung durch Biomasseproduktion und Wärmeerzeugung verwendet.

Somit können die Fassadenelemente 4 mit ihren Bioreaktoren 5 neben der Produktion von Biomasse und Wärme dem Sonnen- und Blendschutz dienen, um den Tageslicht- und Energieeintrag in das Gebäudeinnere zu beeinflussen. Durch die Steuerung der Organismendichte in den Bioreaktoren kann die Licht- und Energiedurchlässigkeit gesteuert werden. Durch die Verstellung der Position und/oder Winkelstellung der Bioreaktoren kann ferner eine gezielte Lichtlenkung in das Gebäudeinnere erfolgen.

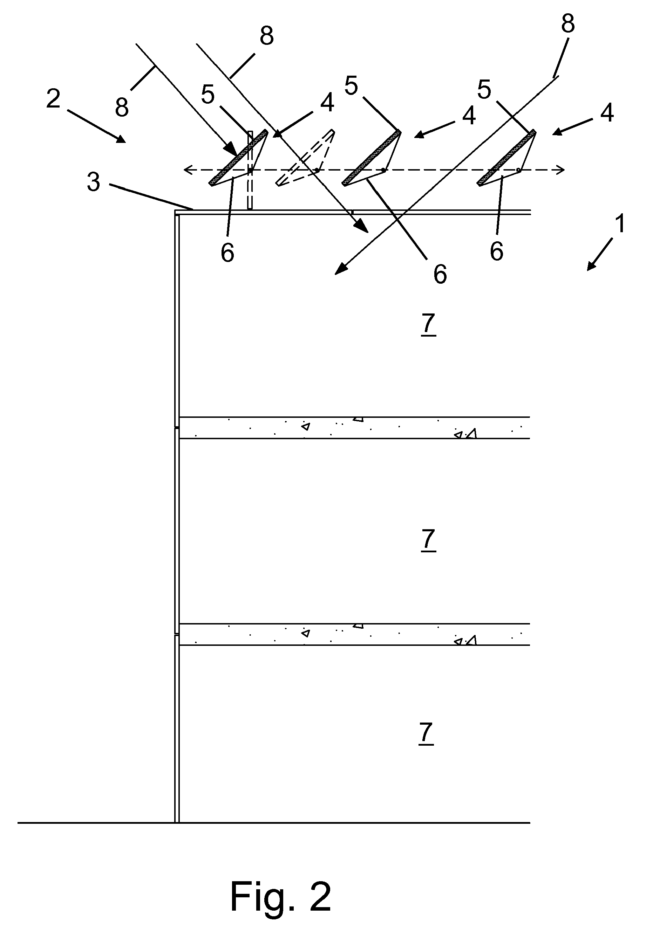

Das in Figur 2 gezeigte Gebäude 1 weist eine Fassade 2 auf einem einen Abschnitt einer Gebäudehülle bildenden Flachdach 3 auf, das teilweise oder vollständig verglast ist. Ansonsten ähnelt die Ausführungsform der Figur 2 der in Figur 1 gezeigten Ausführungsform, d.h. auf dem Dach 3 sind mehrere Fassadenelemente 4 befestigt, die jeweils horizontal entlang des Daches 3 in ihrer Position verfahren und/oder in ihrer Winkelstellung geändert werden können, um variabel mehr oder weniger Sonnenstrahlen 8 zu absorbieren und die genannten Wirkungen zu erzielen.

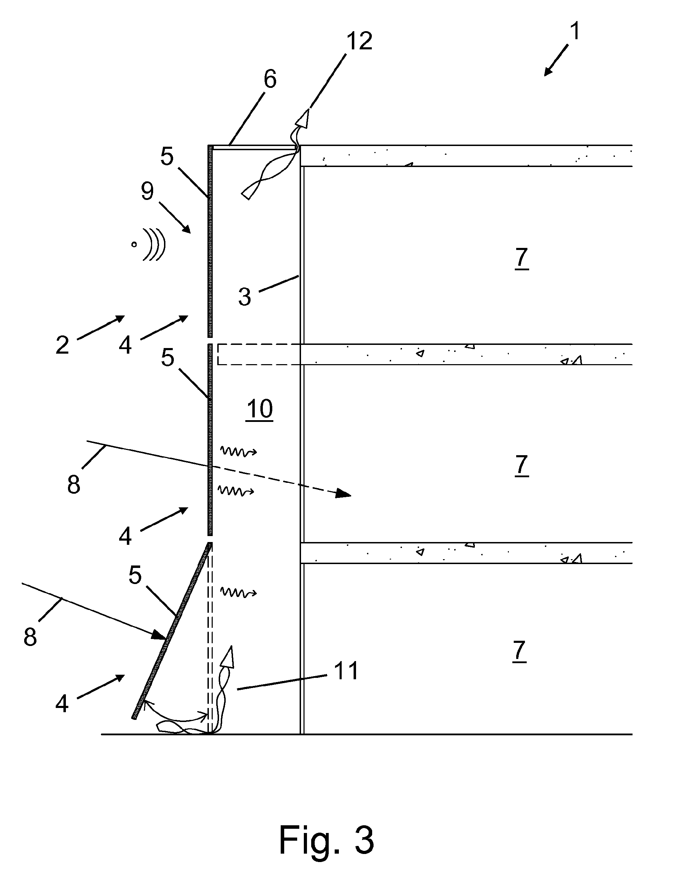

In Figur 3 ist ein Gebäude 1 mit einer zweischaligen Fassade 2 gezeigt. Die Außenschale 9 der Fassade 2 wird durch die plattenförmigen Bioreaktoren 5 einer Vielzahl von Fassadenelementen 4 gebildet, und die Innenschale wird durch eine senkrechte Außenwand 3, d.h. einen Abschnitt der Gebäudehülle, gebildet, an der die Fassadenelemente 4 befestigt sind. Die Außenschale 9 und die Außenwand 3 verlaufen parallel zueinander und sind voneinander beabstandet, so dass zwischen ihnen ein luftgefüllter Zwischenraum 10 existiert, der auch als Nutz- und Erschließungsraum dienen kann. Der Zwischenraum 10 kann dabei klimatische Bedingungen aufweisen, die zwischen dem des Makroklima des Außenraums und dem Miroklima des Innenraumes liegen. Die Bioreaktoren 5 können zusätzlich der Absturzsicherung dienen.

Die einzelnen Bioreaktoren 5 sind in der Weise an einer Haltekonstruktion 6 montiert, dass sie um eine entlang ihres oberen Randes verlaufende horizontale Achse aus der für die beiden oberen Fassadenelemente 4 gezeigten Stellung, in der sie sich parallel zur Außenwand 3 erstrecken, in eine für das untere Fassadenelement 4 gezeigte Stellung geschwenkt werden können, in der sich der Bioreaktor 5 in einem Winkel zur Außenwand 3 erstreckt. Alternativ oder zusätzlich ist es auch möglich, eine Verschwenkbarkeit um eine senkrechte Achse vorzusehen. Durch das Öffnen der Außenschale durch Verschwenken des, in diesem Beispiel, unteren Bioreaktors 5 können Luft und Wärme in den Zwischenraum 10 eintreten, in diesem durch einen Kamineffekt nach oben steigen und am oberen Ende des Zwischenraums 10 aus diesem austreten 12. Dies wird unterstützt durch die auf diesem Weg stattfindende Erwärmung der Luft durch Sonnenstrahlen 8 und durch von den Bioreaktoren 5 abgegebene Wärme (gewellte Pfeile).

Demnach dienen die Bioreaktoren 5 in diesem Ausführungsbeispiel neben der Produktion von Biomasse und Wärme dem Witterungsschutz und dem Sicht-, Sonnen- und Blendschutz, um den Tageslicht- und Energieeintrag in den Zwischenraum 10 zu beeinflussen. Durch die Steuerung der Organismendichte in den Bioreaktoren 5 kann die Licht- und Energiedurchlässigkeit gesteuert werden. Zusätzlich dienen die Bioreaktoren 5 dem Schallschutz, indem sie Außenlärm absorbieren. Als Lüftungselemente ermöglichen sie das kontrollierten Zu- und Abführen von Luft in den Zwischenraum 10. Die Bioreaktoren 5 geben Wärme an den Zwischenraum 10 ab und dienen so in den Übergangsjahreszeiten und im Winter auch als "Aktive Wärmedämmung", um die Wärmeverluste des Zwischenraums 10 zum Außenklima zu reduzieren. Wenn die Bioreaktoren 5 eine geschlossene Haut bilden, kann der Zwischenraum als thermischer Puffer dazu beitragen, die Wärmeverluste des Gebäudes zu reduzieren. Bei geöffneten Bioreaktoren 5 kann vor allem im Sommer erwärmte Luft aus dem Zwischenraum 10 abgezogen werden, um eine natürliche Kühlung des Gebäudes 1 zu unterstützen.

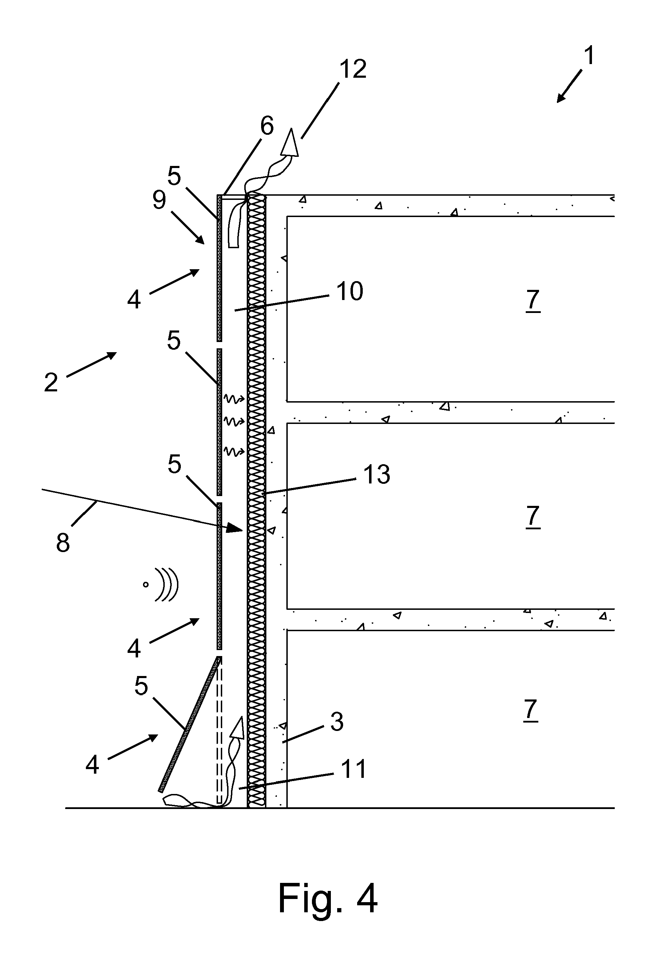

Das in Figur 4 gezeigte Gebäude 1 weist eine Fassade 2 mit einer Ausgestaltung auf, die derjenigen der Figur 3 in Konstruktion und Funktion ähnelt. Allerdings ist der Abstand zwischen der Außenschale 9 und der senkrechten Wandkomponente 3 geringer, und die Fassade 2 stellt eine hinterlüftete Außenwandkonstruktion bereit, deren Außenschale 9 durch verschwenkbare Bioreaktoren 5 gebildet wird. In dem Zwischenraum 10 zwischen der Außenschale 9 und der Wandkomponente 3 befindet sich eine Luftschicht. In der Regel weist die Wandkomponente 3 zur Luftschicht im Zwischenraum 10 hin eine opake oder lichtdurchlässige Dämmebene 13 auf, die auf der Außenseite dunkel- oder hellkaschiert sein kann, um die Absorption von durch die Bioreaktoren 5 gelangender Sonnenenergie 8 zu steuern. In die Außenwandkonstruktion können Fensteröffnungen integriert sein (nicht gezeigt), die zur Belichtung und Belüftung des Innenraumes dienen. Auch in diesem Fall ist es möglich, dass die Bioreaktoren 5 als Absturzsicherung und/oder zur Lichtlenkung dienen.

Die Bioreaktoren 5 dienen hier neben der Produktion von Biomasse und Wärme dem Schlagregenschutz der Außenwandkonstruktion. Zusätzlich ermöglichen sie die Verbesserung des Wärmeschutzes und Schallschutzes der Außenwandkonstruktion. Bei einer geschlossenen Stellung der Bioreaktoren 5 führt die Abgabe von Wärme (gewellte Pfeile) an die Luftschicht im Zwischenraum 10 und der Energieeintrag durch die Bioreaktoren 5 zu einer Erwärmung der Luftschicht, die die Wärmedämmeigenschaften der Wand verbessern. Im Sommer kann durch gezieltes Öffnen im unteren und oberen Bereich der Fassade ein Kamineffekt erzielt werden, der die erwärmten Luftmassen abführt und so zu einer Kühlung der Außenwand beitragen kann.

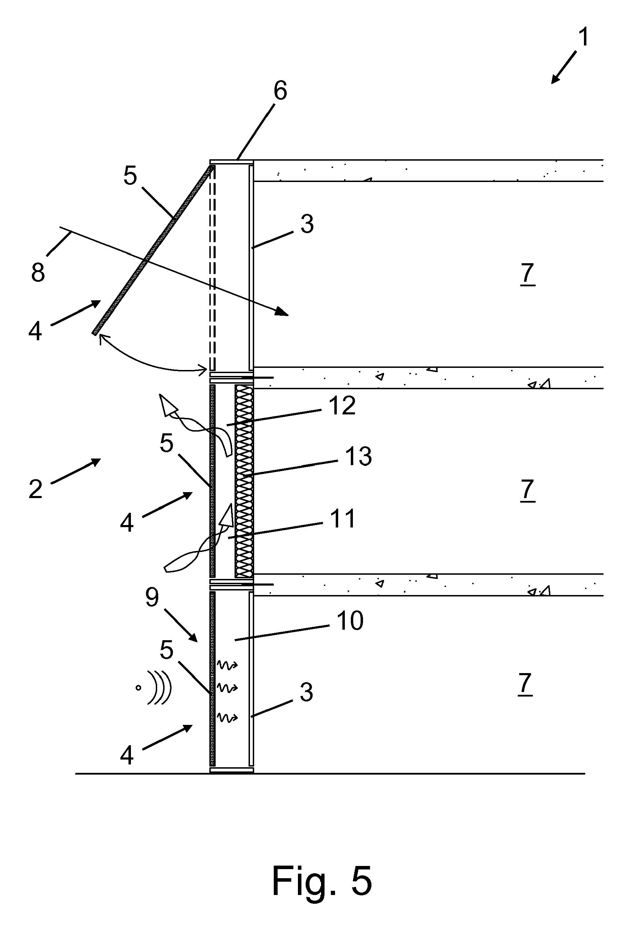

Das in Figur 5 gezeigte Ausführungsbeispiel ähnelt den in den Figuren 3 und 4 gezeigten Ausführungsbeispielen. Der Unterschied besteht darin, dass die einzelnen Fassadenelemente 4 jeweils neben einem plattenförmigen Bioreaktor 5 einen Gebäudehüllen- oder Wandabschnitt 3 aufweisen, der in der für die unteren beiden Fassadenelemente 4 gezeigten Normalstellung parallel zu und beabstandet von dem jeweiligen Bioreaktor 5 verläuft. Dadurch existiert in jedem einzelnen Fassadenelement 4 ein Zwischenraum 10 zwischen einer durch den Bioreaktor 5 gebildeten Außenschale 9 und einer durch den Gebäudehüllenabschnitt 3 gebildeten Innenschale, d.h. jedes Fassadenelement 4 ist für sich zweischalig. Daher können mehrere Fassadenelemente 4 zum Aufbau einer zweischaligen Wand bzw. Fassade verwendet werden. Der Gebäudehüllenabschnitt 3 kann auch ein Kastenfenster sein, dessen äußere Verkleidung durch den zugehörigen Bioreaktor 5 gebildet wird. Der Zwischenraum 10 kann je nach Anwendungsfall groß wie im Ausführungsbeispiel der Figur 3 oder klein wie im Ausführungsbeispiel der Figur 4 gewählt werden.

Die Fassadenelemente 4 können im Werk vorgefertigt und als Fertigkomponenten verwendet oder auf der Baustelle montiert werden. Es sind Fassadenelemente 4 möglich, deren Bioreaktor 5 vor lichtdurchlässigen Bauteilen, wie beispielsweise Verglasungen oder einer lichtdurchlässigen Wärmedämmung, oder opaken, beispielsweise gedämmten Bauteilen 13, vorgesehen ist. Bioreaktoren 5, die vor lichtdurchlässigen Bauteilen wie Verglasungen angeordnet sind, können zusätzlich der Absturzsicherung dienen.

Ähnlich wie für die Ausführungsbeispiele der Figuren 3 und 4 beschrieben ist in jedem Fassadenelement eine Haltekonstruktion vorgesehen, durch die der jeweilige Bioreaktor 5 um eine entlang seines oberen Randes verlaufende horizontale Achse aus der für die beiden unteren Fassadenelemente 4 gezeigten Stellung, in der er sich parallel zum Gebäudehüllenabschnitt 3 erstreckt, in eine für das obere Fassadenelement 4 gezeigte Stellung geschwenkt werden können, in der sich der Bioreaktor 5 in einem Winkel zum Gebäudehüllenabschnitt 3 erstreckt. Alternativ oder zusätzlich ist es auch möglich, eine Verschwenkbarkeit um eine senkrechte Achse vorzusehen. Durch das Öffnen des Bioreaktors 5 durch Verschwenken kann wieder Luft und Wärme in den jeweiligen Zwischenraum 10 eintreten.

Vergleichbar zu den vorhergehenden Fällen dienen die Bioreaktoren 5 neben der Produktion von Biomasse und Wärme dem Schlagregenschutz der Außenwandkonstruktion und dem Sicht-, Sonnen- und Blendschutz, um den Tageslicht- und Energieeintrag in die Zwischenräume 10 zu beeinflussen. Durch die Steuerung der Organismendichte in den Bioreaktoren 5 kann die Licht- und Energiedurchlässigkeit gesteuert werden. Zusätzlich ermöglichen sie die Verbesserung des Wärmschutzes und Schallschutzes der Außenwandkonstruktion. Durch das Schließen und Öffnen der Bioreaktoren 5 kann die Luftschicht- bzw. der Luftraum 10 kontrolliert be- und entlüftet werden. Bei geschlossenen Bioreaktoren 5 trägt die Luftschicht bzw. der Luftraum 10 zur Verbesserung des Wärmeschutzes bei. Bei geöffneten Bioreaktoren 5 können solare Energiegewinne in dem Zwischenraum 10 abgeführt werden. Zusätzlich kann über den Zwischenraum 10 und Lüftungselemente in der Innenschale das Gebäudeinnere be- und entlüftet werden.

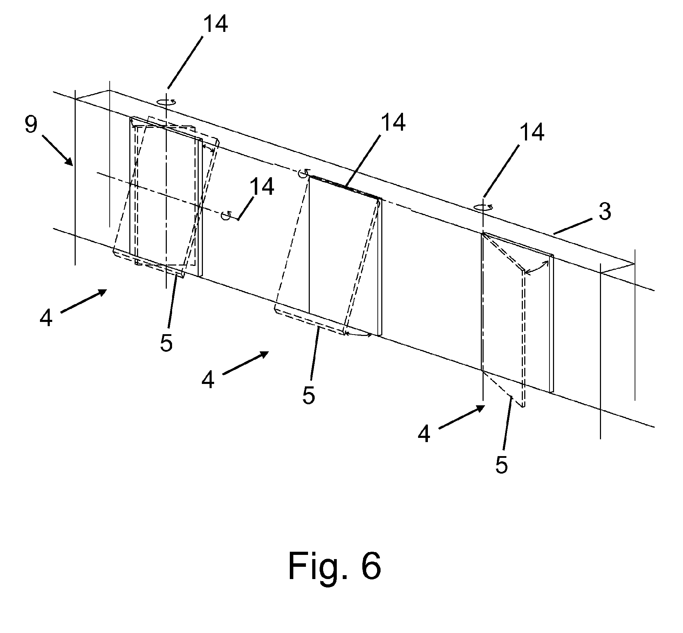

Figur 6 zeigt verschiedene Möglichkeiten der schwenkbaren Befestigung von Bioreaktoren 5 in der Außenschale einer Fassade. Im linken Fall ist der Bioreaktor 5 um sowohl eine waagerechte als auch eine senkrechte Achse 14 schwenkbar gelagert, die jeweils durch das Zentrum des Bioreaktors 5 verlaufen. Im mittleren Fall ist der Bioreaktor 5 um eine horizontale Achse 14 schwenkbar gelagert, die sich entlang seines oberen Randes erstreckt, und im rechten Fall ist der Bioreaktor 5 um eine senkrechte Achse 14 schwenkbar gelagert, die sich entlang seines linken Randes erstreckt.

## Patentansprüche

1. Fassadenelement mit einem plattenförmigen Bioreaktor (5) zur Kultivierung phototropher Organismen, der ein lichtdurchlässiges plattenförmiges Gehäuse aufweist, in dessen Innerem ein Reaktorraum zur Aufnahme der phototrophen Organismen ausgebildet ist.

2. Fassadenelement nach Anspruch 1, das ferner eine Befestigungseinrichtung (6) zur Befestigung des Bioreaktors (5) an einer Außenseite eines Gebäudes (1) aufweist.

3. Fassadenelement nach Anspruch 2, bei dem die Befestigungseinrichtung (6) in der Weise ausgestaltet ist, dass der Bioreaktor (5) nach Befestigung an einem Gebäudehüllenabschnitt (3) eines Gebäudes (1) mit Hilfe der Befestigungseinrichtung (6) von dem Gebäudehüllenabschnitt (3) beabstandet ist und sich zwischen dem Bioreaktor (5) und dem Gebäudehüllenabschnitt (3) ein Zwischenraum (10) befindet.

4. Fassadenelement nach Anspruch 1 oder Anspruch 2, das einen Gebäudeschalenabschnitt (3) aufweist, an dem der Bioreaktor (5) in der Weise befestigt ist, dass er von dem Gebäudehüllenabschnitt (3) beabstandet ist und sich zwischen dem Bioreaktor (5) und dem Gebäudehüllenabschnitt (3) ein Zwischenraum (10) befindet.

5. Fassadenelement nach einem der vorhergehenden Ansprüche, das ferner eine Verstelleinrichtung aufweist, mit der eine Position und/oder Winkelstellung des Bioreaktors (5) nach Befestigung an einer Außenfläche eines Gebäudes (1) in Bezug auf das Gebäude (1) verändert werden kann.

6. Fassadenelement nach einem der vorhergehenden Ansprüche, bei dem das Fassadenelement (4) einen Anschluss aufweist, an den eine Wärmetransporteinrichtung angeschlossen werden kann, um Wärme zwischen dem Bioreaktor (5) und einem Gebäude (1) auszutauschen.

7. Fassadenelement nach Anspruch 6, bei dem in dem Bioreaktor (5) eine Leitung angeordnet ist, die in Bezug auf den Reaktorraum so angeordnet und ausgestaltet ist, dass im Betrieb zwischen einem die Leitung durchströmenden Fluid und einem in dem Reaktorraum angeordneten Kulturmedium Wärme ausgetauscht werden kann, wobei zwei gegenüberliegende Enden der Leitung mit dem Anschluss verbunden sind, so dass über den Anschluss Fluid in die Leitung einströmen und aus dieser wieder austreten kann, nachdem es die Leitung durchströmt hat.

8. Fassadenelement nach einem der vorhergehenden Ansprüche, bei dem dem Fassadenelement eine Transmissionssteuereinrichtung zugeordnet ist, die angepasst ist, um die Dichte der phototrophen Organismen im Reaktorraum des Bioreaktors (5) gezielt zu verändern und auf diese Weise die Lichtdurchlässigkeit des Bioreaktors (5) einzustellen.

9. Fassadenkonstruktion, die mindestens ein Fassadenelement (4) nach einem der vorhergehenden Ansprüche aufweist.

10. Fassadenkonstruktion nach Anspruch 9, die mindestens zwei nebeneinander angeordnete Fassadenelemente (4) nach einem der Ansprüche 1 bis 8 aufweist, die in Kombination einen durchgehenden Abschnitt der Außenfläche (9) der Fassadenkonstruktion (2) bilden.

11. Gebäude mit mindestens einem Fassadenelement (4) nach einem der Ansprüche 1 bis 8, das an einer Außenseite des Gebäudes (1) befestigt ist, oder einer Fassadenkonstruktion (2) nach einem der Ansprüche 9 und 10.

12. Gebäude nach Anspruch 11, bei dem eine Vielzahl von Fassadenelementen (4) nach einem der Ansprüche 1 bis 8 in der Weise an einer Außenseite des Gebäudes (1) angeordnet ist, dass die Bioreaktoren (5) der Fassadenelemente (4) zumindest einen Teil einer Außenschale (9) des Gebäudes (1) bilden, wobei diese Außenschale (9) in einem Abstand zu einer Gebäudehülle (3) angeordnet sind, so dass zwischen der Gebäudehülle (3) und der Außenschale (9) ein Zwischenraum (10) besteht.

13. Gebäude nach Anspruch 11 oder Anspruch 12, bei dem in einem Gebäudehüllenabschnitt (3) mindestens ein Fenster, eine Tür oder ein verglaster Abschnitt angeordnet ist und bei dem mindestens ein Fassadenelement (4) nach einem der Ansprüche 1 bis 8 an dem Gebäudehüllenabschnitt (3) befestigt und in der Weise in Bezug auf das Fenster, die Tür bzw. den verglasten Abschnitt angeordnet ist und/oder in der Position verfahren werden kann, dass es als variabel einstellbarer Sonnenschutz verwendet werden kann.

14. Gebäude nach einem der Ansprüche 11 bis 13, das eine Wärmetransportvorrichtung aufweist, die an den Anschluss von mindestens einem Fassadenelement (4) nach einem der Ansprüche 1 bis 8 angeschlossen ist, so dass Wärme zwischen den Bioreaktoren (5) der jeweiligen Fassadenelemente (4) und dem Gebäude (1) ausgetauscht werden kann.

15. Gebäude nach Anspruch 14, das eine Wärmespeicherungseinrichtung aufweist, die mit der Wärmetransportvorrichtung funktional in der Weise gekoppelt ist, dass Wärme aus den Bioreaktoren (5) von Fassadenelementen (4), an die die Wärmetransportvorrichtung angeschlossen ist, in der Wärmespeicherungseinrichtung gespeichert werden kann und die Wärmespeicherungseinrichtung Wärme an die Bioreaktoren (5) abgeben kann.
